# EUROPEAN PATENT APPLICATION

(11) **EP 4 803 076 A1**
(43) Date of publication of application: **09.09.2026**
(21) Application number: 25161437.6
(22) Date of filing: 03.03.2025
(51) Int. Cl.: A61K 9/70, A61K 47/12, A61K 38/26

(54) **TRANSMUCOSAL THERAPEUTIC SYSTEM CONTAINING A GLP-1 RECEPTOR AGONIST**

(71) Applicant: LTS Lohmann Therapie-Systeme AG, 56626 Andernach (DE)
(72) Inventor: MOHR, Patrick, 53498 Bad Breisig (DE); LODDER-GADACZEK, Julia, 65618 Selters (DE)
(74) Representative: Maiwald GmbH

(57) **Abstract**

The present invention relates to transmucosal therapeutic systems for the transmucosal administration of a GLP-1 receptor agonist comprising a mucoadhesive layer structure comprising a therapeutically effective amount of a GLP-1 receptor agonist, such GLP-1 receptor agonist-containing transmucosal therapeutic systems for use in a method of treatment, a method of treatment comprising applying such GLP-1 receptor agonist-containing transmucosal therapeutic systems, and processes of manufacture of such transmucosal therapeutic systems.

## Description

### TECHNICAL FIELD OF THE INVENTION

The present invention relates to a transmucosal therapeutic system for the transmucosal administration of a GLP-1 receptor agonist to the systemic circulation, and processes of manufacture, methods of treatment and uses thereof.

### BACKGROUND OF THE INVENTION

GLP-1 receptor agonists (also known as GLP-1 agonists, GLP-1 analogs, or incretin mimetics) represent a class of medications used in treating type 2 diabetes mellitus (T2DM) and obesity.

The GLP-1 receptor is a core member of the G protein-coupled receptor (GPCR) family and is widely present on the surfaces of various cell types within the human body. It specifically interacts with glucagon-like peptide-1 (GLP-1), a key hormone that plays an integral role in regulating blood glucose levels, lipid metabolism, and several other crucial biological functions. GLP-1 receptor agonists are synthetic protein preparations that have partial or complete amino acid sequence homology with endogenous GLP-1, but are less easily degraded and have longer half-lives. By emulating and even exceeding the biological activity of natural GLP-1, GLP-1 receptor agonists effectively fulfill the role of GLP-1 and are thus able to enhance insulin secretion, inhibit glucagon release, delay gastric emptying, and reduce food intake through central appetite suppression.

In recent years, GLP-1 medications have gained significant attention in the medical community due to their innovative treatment mechanisms, significant therapeutic efficacy, and broad development prospects. Starting with the U.S. Food and Drug Administration (FDA) approval of exenatide in 2005, therapies based on GLP-1 receptor agonists have become the treatment of choice for T2DM and obesity. FDA-approved GLP-1 receptor agonists for glycemic control include dulaglutide, exenatide, liraglutide, lixisenatide, semaglutide and tirzepatide. Furthermore, liraglutide, semaglutide and tirzepatide are FDA-approved as pharmacologic treatment for weight loss.

Liraglutide (manufactured by Novo Nordisk) was first approved by the FDA in 2010 under the brand name Victoza^{®} for the treatment of T2DM and was FDA-approved for other indications, such as chronic weight management, under the brand name Saxenda^{®} in 2014. Semaglutide (also manufactured by Novo Nordisk) is sold as anti-diabetic medication used for the treatment of T2DM under the brand names Ozempic^{®} (FDA-approved in 2017) and Rybelsus^{®} (FDA-approved in 2019) and anti-obesity medication used for long-term weight management under the brand name Wegovy^{®} (FDA-approved in 2021). Tirzepatide (manufactured by Lilly) is the first dual agonist (twincretin), activating the gastric inhibitory peptide (GIP) receptor in addition to the GLP-1 receptor, and is thus considered by the FDA to be a first-in-class medication. It was approved by the FDA for treating T2DM under the brand name Munjaro^{®} in 2022 and for treating obesity under the brand name Zepbound^{®} in 2023.

The above GLP-1 medications are typically provided as injectable solutions in a prefilled pen, as the poor oral bioavailability of GLP-1 receptor agonists requires subcutaneous administration. As for semaglutide, an oral dosage form is also available (Rybelsus^{®}). The oral semaglutide absorption is enabled by using a tablet formulation comprising salcaprozate sodium (SNAC) as permeation enhancer. However, the oral bioavailability of semaglutide remains poor and is described to be less than 1%. Accordingly, the tablets are for daily administration, providing dosages of 3 mg (initial dose), 7 mg (maintenance dose) or 14 mg (maximum dose). In contrast, an injectable dosage form of semaglutide (Ozempic^{®}) provide dosages of 0.25 mg (initial dose), 0.5 mg (maintenance dose) and 1 or 2 mg (maximum dose) and only need to be administered once weekly.

In general, patients tend to prefer less frequent dosing regimens, as well as oral dosage forms over injectable medication, in particular as they do not require the use of needles (needle phobia) and do not incite injection-site reactions such as pruritus and erythema. However, the high drug load needed to compensate the poor bioavailability of GLP-1 receptor agonists in oral dosage forms results in an increased potential of adverse effects. The most frequently exhibited adverse effects from GLP-1 receptor agonists are gastrointestinal and include nausea, vomiting, and diarrhea that could lead to an acute kidney injury due to volume contraction. Severe to life-threatening adverse effects further include pancreatitis, hypoglycemia, thyroid tumors and cancer, visual disturbances (diabetic retinopathy) and allergic reactions, while mild to moderate adverse effects such as dizziness, mild tachycardia, headaches, dyspepsia including constipation and taste disturbances may also occur.

There is thus a need for other dosage forms which are more convenient for the patient and preferably may also reduce the occurrence of adverse effects.

For example, the disadvantages of oral and also subcutaneous dosage forms could be reduced by using a transmucosal therapeutic system that releases sufficient amount of a GLP-1 receptor agonist within a reasonable period of application of, e.g., about 30 or about 45 minutes, or about 1 or about 2 hours. The transmucosal administration route is a less common but very attractive alternative route compared to oral administration, since it is non-invasive and provides for the possibility of self-administration. An active agent administered transmucosally reaches systemic circulation by absorption *via* mucosal tissue directly, which enables a rapid onset of action and bypasses the first-pass metabolism, thus preventing the degradation, metabolization and potentially low absorption due to the gastrointestinal passage of the oral administration route. Transmucosal administration encompasses intranasal, oral as well as rectal administration, of which in terms of ease of administration, the oral transmucosal route, also termed oromucosal route, is preferred.

Formulating appropriate dosage forms for the transmucosal delivery is challenging due to a multitude of aspects to be considered and issues to be solved. The main requirements for such transmucosal therapeutic systems are good adhesion and active permeation, combined with an appropriate behavior and time of disintegration. In the present case, as peptides having a molecular weight of 3,000 g/mol or more (e.g., Liraglutide has 3,751 g/mol, Semaglutide has 4,114 g/mol, and Tirzepatide has 4,813 g/mol) are supposed to permeate transmucosally, appropriate permeation enhancers need to be found in order to allow delivery of GLP-1 receptor agonists throughout the oral mucosa. Up to date, no commercial GLP-1 receptor agonist transmucosal therapeutic system is available.

It is therefore desirable to provide a transmucosal therapeutic system for the transmucosal administration of a GLP-1 receptor agonist, which is suitable to replace the current (subcutaneous and oral) dosage forms and to be used for the treatment of T2DM and/or obesity.

### OBJECTS AND SUMMARY OF THE INVENTION

It is an object of the present invention to provide a transmucosal therapeutic system overcoming the above-mentioned disadvantages of current administration of GLP-1 receptor agonists.

Thus, it is an object of the present invention to provide a transmucosal therapeutic system for the transmucosal administration of a GLP-1 receptor agonist providing a permeation rate which is sufficient for achieving a therapeutically effective dose. In particular, the object is to provide a transmucosal therapeutic system for the transmucosal administration of a GLP-1 receptor agonist, providing therapeutically effective amounts of the GLP-1 receptor agonists within less than 5 hours, such as within less than 3 hours, within less than 2 hours, within less than 1.5 hours or 90 minutes. Longer periods of application allowing dosing intervals such as at least 24 hours, 48 hours, or even a twice or once-weekly exchange may also be desirable depending on the situation.

It is a further an object of the present invention to provide a transmucosal therapeutic system for the transmucosal administration of a GLP-1 receptor agonist providing a total amount permeated of the GLP-1 receptor agonist per period of application which corresponds to the required daily dose of current administration at a reasonable patch size. In particular, the object is to provide a transmucosal therapeutic system for the transmucosal administration of a GLP-1 receptor agonist having an area of release of not more than 10 cm², or not more than 5 cm², releasing at least 10 µg, at least 50 µg or at least 100 µg, of the GLP-1 receptor agonist per period of application.

It is a further object of the present invention to provide a transmucosal therapeutic system for the transmucosal administration of a GLP-1 receptor agonist with a high active ingredient utilization. In particular, the object is to provide a transmucosal therapeutic system for the transmucosal administration of a GLP-1 receptor agonist, releasing at least 1 %, at least 5 % or at least 10 % of the active ingredient contained therein within about 5 hours after application, or within about 3 hours after application.

It is further an object of the present invention to provide a transmucosal therapeutic system for the transmucosal administration of a GLP-1 receptor agonist which complies with the needs of a convenient application and handling in view of dimensions, provides for good patient compliance and/or which is easy and cost-efficient to manufacture.

It is a further object of the present invention is to provide a transmucosal therapeutic system for the transmucosal administration of a GLP-1 receptor agonist providing appropriate adhesion to the mucosa, e.g. initially but also over time.

It is a further object of the present invention is to provide a transmucosal therapeutic system for the transmucosal administration of a GLP-1 receptor agonist providing appropriate disintegration behavior, e.g. in terms of the disintegration time but also in terms of integrity of the transmucosal therapeutic system.

It is a further object of the present invention is to provide a transmucosal therapeutic system for the transmucosal administration of a GLP-1 receptor agonist having an acceptable flavor profile, in particular in order to avoid or alleviate unpleasant olfactory-gustatory sensations which might be caused by any of the ingredients contained therein.

It is also a further object of the present invention to provide a transmucosal therapeutic system for the transmucosal administration of a GLP-1 receptor agonist which does not provoke an irritating sensation at the mucosa or otherwise in the oral cavity.

It is another object of the present invention to provide a transmucosal therapeutic system for the transmucosal administration of a GLP-1 receptor agonist which can be used in a method of treatment.

These objects and others are accomplished by the present invention, which according to one aspect relates to a transmucosal therapeutic system for the transmucosal administration of a GLP-1 receptor agonist comprising a mucoadhesive layer structure, said mucoadhesive layer structure comprising
A) an active agent-containing layer comprising
   1. a GLP-1 receptor agonist;
   2. a combination of permeation enhancers; and
   3. a dissolvable film-forming agent, wherein
the combination of permeation enhancers comprises
i) a first permeation enhancer selected from the group consisting of C6 to C12 fatty acids and salts thereof; and
ii) a second permeation enhancer selected from the group consisting of bile acids and salts thereof.

It has been surprisingly found that the transmucosal therapeutic system according to the present invention, which comprises in the active agent-containing layer a GLP-1 agonist together with a combination of permeation enhancers comprising a C6 to C12 fatty acid or a salt thereof as first permeation enhancer and a bile acid or a salt thereof as second permeation enhancer is advantageous in terms of permeation properties and active ingredient utilization. The enhancer combination is preferably sodium caprate and sodium glycocholate. In particular, it has been found that the enhancer combination allows for sufficient release of GLP-1 receptor agonists (such as, e.g., 10 µg or more per application) within a reasonable period of application (such as, e.g., 2 hours or less), enabling the resulting transmucosal therapeutic system to comprise an acceptable content of the GLP-1 receptor agonist (such as, e.g., 1 mg/cm² or less) and to have comfortable dimensions, in particular with regard to the area of release (such as, e.g., 10 cm² or less) and the area weight of the active agent-containing layer (such as, e.g., 250 g/m² or less).

According to one specific aspect, the present invention relates to transmucosal therapeutic system for the transmucosal administration of a GLP-1 receptor agonist comprising a mucoadhesive layer structure, said mucoadhesive layer structure comprising at least
A) an active agent-containing layer comprising
   1. a GLP-1 receptor agonist in an amount of from 2 wt-% to less than or equal to 10 wt-%;
   2. a combination of permeation enhancers in an amount of from 12 wt-% to less than or equal to 36 wt-%; and
   3. a dissolvable film-forming agent in an amount of from 40 wt-% to less than or equal to 60 wt-%, wherein

the combination of permeation enhancers comprises
   i) a first permeation enhancer selected from the group consisting of C6 to C12 fatty acids and salts thereof; and
   ii) a second permeation enhancer selected from the group consisting of bile acids and salts thereof, and
the dissolvable film-forming agent is a hydroxypropyl methyl cellulose.

According to certain embodiments of the invention, the transmucosal therapeutic system according to the invention is for use in a method of treating a human patient, such as in a method of treating diabetes, in particular type 2 diabetes mellitus, and/or in a method of treating obesity, in particular in a method of supporting weight management, including weight loss or weight maintenance.

According to certain embodiments of the invention, the present invention relates to the use of a transmucosal therapeutic system according to the invention for the manufacture of a medicament for treating a human patient, such as for treating diabetes, in particular type 2 diabetes mellitus, and/or for treating obesity, in particular for supporting weight management, including weight loss or weight maintenance.

According to certain embodiments of the invention, the invention relates to a method of treating a human patient, such as a method of treating diabetes, in particular type 2 diabetes mellitus, and/or a method of treating obesity, in particular a method of supporting weight management, including weight loss or weight maintenance, including administering a transmucosal therapeutic system according to the invention to a human patient.

According to another aspect, the present invention relates to a process manufacture of an active agent-containing layer of a transmucosal therapeutic system according to the invention, comprising the steps of:
(a) combining at least the GLP-1 receptor agonist, the combination of permeation enhancers and the dissolvable film-forming agent in a solvent to obtain a coating composition;
(b) coating the coating composition onto a release liner; and
(c) drying the coated coating composition to form the active agent-containing layer.

### DEFINITIONS

Transmucosal therapeutic systems, or transmucosal delivery systems (also termed buccal patches by some), consist of one or more thin layers which are applied and adhere to the mucosa, in particular of the oral cavity, to deliver the drug over a period of time. Dosage forms in the form of thin films for application in the oral cavity are also sometimes referred to as "Oral Thin Film" or OTF, however, OTFs are not necessarily intended to adhere to the mucosa. In a transmucosal therapeutic system, the active is contained in a dissolvable layer, and due to the film adhering to the mucosa, active delivery is achieved by a combination of direct active release from the transmucosal therapeutic system to the mucosa, and by an indirect active delivery *via* dissolution in the saliva.

Within the meaning of this invention, the term "transmucosal therapeutic system" or "transmucosal delivery system" refers to a system by which the active agent (GLP-1 receptor agonist) is administered to the systemic circulation *via* transmucosal delivery by application to the mucosa such as of the oral cavity, and refers to the entire individual dosing unit that is applied to the mucosa of a patient, and which comprises a therapeutically effective amount of the GLP-1 receptor agonist in a mucoadhesive layer structure and optionally an additional overlay on top of the active agent-containing mucoadhesive layer structure. Specific transmucosal therapeutic systems which are applied to the mucosa of the oral cavity are also referred to as oromucosal therapeutic systems. The mucoadhesive layer structure may be located on a release liner (a detachable protective layer), thus, the transmucosal therapeutic system may further comprise a release liner. In the sense of the invention, the term "transmucosal therapeutic system" is in particular understood to mean a system providing passive transmucosal delivery excluding active transport as in methods including microporation. Also, in contrast to certain oral thin films which are not necessarily mucoadhesive and which are intended to disintegrate very fast in the saliva (sometimes referred to as "flash wafers"), enteral delivery is entirely unintended in transmucosal therapeutic systems.

Within the meaning of this invention, the term "mucoadhesive layer structure" or "mucoadhesive layer structure containing a therapeutically effective amount of the GLP-1 receptor agonist" refers to the active agent-containing structure providing the area of release for the GLP-1 receptor agonist during administration. Any additional overlay adds to the overall size of the transmucosal therapeutic system but does not add to the area of release. The mucoadhesive layer structure comprises at least one active agent-containing layer.

Within the meaning of this invention, the term "therapeutically effective amount" refers to a quantity of active agent in the transmucosal therapeutic system sufficient to provide, if administered by the transmucosal therapeutic system to a patient, GLP-1 receptor agonist blood levels of a similar range (e.g. of about 10 % to about 1000 % as measured as an AUC) when compared to blood levels obtained in a one-time administration of commercially available approved drug products of the active agent, such as 0.5-8 mg subcutaneous semaglutide or 1.5-14 mg oral semaglutide.

Within the meaning of this invention, the terms "active", "active agent", and the like, as well as the term "GLP-1 receptor agonist" refer to GLP-1 receptor agonists in any pharmaceutically and/or regulatorily acceptable chemical and morphological form and physical state. These forms include without limitation GLP-1 receptor agonists in their free, dissociated or any associated form such as hydrates, solvates and so on, as well as GLP-1 receptor agonists in the form of particles. In accordance with the invention, the term "particles" is understood to mean to a solid, particulate material comprising individual particles, the dimensions of which are negligible compared to the material. In particular, the particles are solid, including plastic/deformable solids, including amorphous and crystalline materials.

The GLP-1 receptor agonist, where contained in a medium such as a solvent, may be dissolved or dispersed or in part dissolved and in part dispersed. In the sense of the invention, the term "dispersing" is understood to mean a step or a combination of steps wherein a starting material is not totally dissolved. Dispersing in the sense of the invention comprises the dissolution of a part of the starting material, depending on the solubility of the starting material (e.g. the solubility of the GLP-1 receptor agonist in the coating composition).

Within the meaning of this invention, the terms "GLP-1 receptor agonist", "GLP-1 agonist" and "GLP-1 analog" refer to a group of active agents mimicking the actions of the endogenous incretin hormone GLP-1. GLP-1 is a 30-amino acid polypeptide processed from proglucagon in the endocrine L-cells distributed primarily in the mucosa of the distal part of the small intestine and colon. Together with GIP (glucose-dependent insulinotropic polypeptide), the most import effect of GLP-1 is its ability to potentiate glucose-induced insulin secretion from the pancreas, the so-called incretin effect. Thus, GLP-1 receptor agonists are also referred to as "incretin mimetics". GLP-1 receptor agonists are usually (synthetic) peptides or peptide conjugates, resulting from intricate structural modifications to GLP-1, enabling them to not only replicate the pharmacological functions of GLP-1 but also to render them resistant to degradation which resulting in a prolonged half-life and heightened biological activity. These synthetic protein preparations can exhibit partial or complete amino acid sequence identity with endogenous GLP-1 (in particular of human GLP-1(7-37), the amino acid sequence of which is HAEGTFTSDVSSYLEGQAAKEFIAWLVKGRG). For example, liraglutide shares 97% sequence identity with human GLP-1 (compared to GLP-1(7-37) the lysine residue at position 34 is replaced by arginine and a hexadecanoyl group is attached to the remaining lysine at position 26 via a spacer), and semaglutide shares 94% sequence identity with human GLP-1 (compared to GLP-1(7-37) the alanine residue at position 8 is replaced by diaminoisobutyric acid, the lysine residue at position 34 is replaced by arginine and a stearic diacid chain is attached to the remaining lysine at position 26 via a spacer). In comparison, tirzepatide has less sequence identity to human GLP-1. It is derived from the native sequences of GLP-1, GIP and semaglutide with the incorporation of further distinct residues. As a result, it serves as a dual agonist for both GLP-1 and GIP. Accordingly, the term "GLP-1 receptor agonist" also relate to dual agonists, targeting both the GLP-1 receptor and another specific receptor, such as the GIP receptor, or to triple agonist, simultaneously targeting the GLP-1 receptor and two other specific receptors. Furthermore, the term "GLP-1 receptor agonists" includes small molecules acting as GLP-1 receptor agonists, such as, e.g., orforglipron or danuligpron.

Within the meaning of the invention, the term "sequence identity" refers to the degree of identity of a first amino acid sequence to a second amino acid sequence, and is calculated as a percentage based on a comparison between the two sequences. The sequence identity is determined by a program, which produces a pairwise alignment, and calculates the identity between the two aligned sequences counting both mismatches at a single position and gaps at a single position as non-identical positions. Sequence identity can be calculated from a pairwise alignment of two sequences over the full length of both sequences ("global sequence identity"), or from a pairwise alignment of the local regions of the first sequence and the second sequence that show identity or similarity ("local sequence identity"). If not indicated otherwise, a sequence identity within the meaning of this invention refers to a sequence identity that is calculated from a pairwise alignment taken into account human GLP-1(7-37) and the sequence of the GLP-1 receptor agonist over its full length. An exemplary program for determining a "global sequence identity" is the "Needle" (The European Molecular Biology Open Software Suite, EMBOSS) program (https://www.ebi.ac.uk/Tools/psa/emboss_needle/). Alignments showing the "local sequence identity" can, for example, be produced by the Blast algorithm (NCBI).

Within the meaning of the invention, the term "peptide" refers to a primary sequence of amino acids that are linked by covalent "peptide linkages." In general, a peptide consists of fewer amino acids than a full-length protein, and typically comprises from 2-50 amino acids. A synthetic peptide is a peptide that is produced by artificial means *in vitro* (e.g., was not produced in vivo). A peptide amino acid sequence may also comprise chemical compounds (peptide conjugate). As used herein, the term "amino acid sequence," refers to the primary (i.e., linear) structure of a peptide or protein, wherein the individual amino acids are linked by peptide bonds.

There are two main types of transmucosal therapeutic systems, i.e. those using backing layers, and those without. Active delivery of an open system type transmucosal therapeutic system using no backing layer will always be a combination of direct delivery from the transmucosal therapeutic system through the mucosa at the adhesion site, and indirect delivery *via* dissolution of the active from the transmucosal therapeutic system into the saliva, and from the saliva through the mucosa. The proportion of the different delivery routes depends mainly on factors such as the solubility of the active and the disintegration time of the transmucosal therapeutic system. The higher the solubility and the faster disintegration of the transmucosal therapeutic system, dissolution into the saliva will be favored over direct delivery into the mucosa at the adhesion site. Such an indirect delivery has the huge advantage of providing a practical increase by several factors of the mucosal surface area through which the active is released systemically. Dissolution into the saliva on the other hand means that the active concentration, and thus the final delivered amount is difficult to control, and that there may be a risk of enteral delivery by unintended swallowing of the saliva.

Transmucosal therapeutic systems using a backing layer have a completely different approach, i.e. in such systems, the loss of being restricted in the drug release area (to the actual size of the patch) is accepted in exchange for limiting the delivery route to the direct transmucosal delivery, which can be much better controlled. Thus, in the sense of the invention, a "backing layer" is any layer within a transmucosal therapeutic system which is able to prevent (at least a substantial amount of) the active contained within the transmucosal therapeutic system to be dissolved into the saliva. Such a backing layer can be non-dissolvable, or dissolvable over time. In the latter case, the time the backing layer takes for dissolution is at least as long as (a substantial amount of) the active takes to be delivered through the mucosa.

In this context, it also becomes clear that terms such as "dissolution", "dissolvable", "dissolve" and the like with respect to any of the layers of a transmucosal therapeutic system (e.g. backing layer, active agent-containing layer) and with respect to the film-forming agent when casted into a film, are to be understood very broadly, and not in the strict scientific sense of chemically dissolving a molecule in a solvent. Any transformation of the solid state of the layer concerned to a liquid state, such as dispersing, forming of a suspension, gelling of the film and disintegrating into smaller parts of gel, etc. has to be regarded as "dissolving" in the sense of the present invention, as long as the "dissolved" material is able to freely move around in the liquid (e.g. saliva) so that anything that was present below the layer concerned (i.e. the mucosa if a mucosa-contacting layer was dissolved, or e.g. the active agent-containing layer if a backing layer was dissolved before the active agent-containing layer) becomes accessible to liquid other than the "dissolved" material. In preferred embodiments, the meaning is limited to the usual chemical sense of dissolving a molecule in a solvent. It should be noted that the term "dissolve" with respect to substances *per se,* such as the active agent, or any excipients, will continue to be used in the usual chemical sense of dissolving a molecule in a solvent. E.g., active agent in dissolved form obviously does not include active agent in dispersed form. The film-forming agent *per se* can be present in the coating composition during manufacture of the transmucosal therapeutic system in dissolved form in the common chemical sense (e.g. is not dispersed, in form of small parts of gel, etc.), but where the film-forming agent is casted into a film, "dissolving" such a film also includes gelling of the film and disintegrating into smaller parts of gel.

Within the meaning of the invention, the term "active agent-containing layer" refers to a layer containing the active agent and providing the area of release. As used herein, the active agent-containing layer is the final, solidified layer e.g. obtained after coating and drying the solvent-containing coating composition. The active agent-containing layer may also be manufactured by laminating two or more such solidified layers (e.g. dried layers) of the same composition to provide the desired area weight. The active agent-containing layer may be mucoadhesive (in the form of a mucoadhesive layer) or the transmucosal therapeutic system may comprise an additional mucosa-contacting layer of a mucoadhesive for providing sufficient adhesion. In particular, the active agent-containing layer is a mucoadhesive layer.

Within the meaning of this invention, the term "mucoadhesive" refers to a material that in particular adheres to and upon contact with a mucosa, but which preferably is non-tacky and can be touched e.g. with the fingers and manipulated, e.g. for application into the oral cavity, without unintentionally adhering to the skin of the fingers, when in dry state. A mucoadhesive layer, when in contact with the mucosa, is "self-adhesive", i.e. provides adhesion to the mucosa so that typically no further aid for fixation is needed. The adhesion strength is preferably strong enough that typical movements in the oral cavity are not sufficient to displace a mucoadhesive layer adhered to the mucosa. A "mucoadhesive" layer structure includes a mucoadhesive layer for mucosa contact which may be provided in the form of a mucoadhesive active agent-containing layer or in the form of an additional layer, i.e. a mucoadhesive mucosa-contacting layer. A mucoadhesive overlay may still be employed to advance adhesion.

Within the meaning of this invention, the term "mucoadhesive overlay" refers to a mucoadhesive layer, which is located on top of the active agent-containing mucoadhesive layer structure, free of active agent, larger in area than the active agent-containing structure and which provides additional area adhering to the mucosa, but no area of release of the active agent. It enhances thereby the overall adhesive properties of the transmucosal therapeutic system.

Within the meaning of this invention, the term "mucosa-contacting layer" refers to a layer included in the transmucosal therapeutic system to be in direct contact with the mucosa of the patient during administration. When the transmucosal therapeutic systems comprises a mucosa-contacting layer, the other layers do not contact the mucosa and do not necessarily have mucoadhesive properties. The area of release is provided by the area of the active agent-containing layer. A mucosa-contacting layer may be used to enhance adherence. The sizes of an additional mucosa-contacting layer and the active agent-containing layer are usually coextensive and correspond to the area of release.

Within the meaning of this invention, the term "area weight" refers to the dry weight of a specific layer, e.g. of the active agent-containing layer, provided in g/m², as determined after normal drying (expecting a residual moisture content of less than about 10% such as in a range of from about 5% to about 8%) or, preferably, after total drying (expecting a residual moisture content of approx.. 0%). The area weight values are subject to a tolerance of ± 10 %, preferably ± 7.5 %, due to manufacturing variability.

If not indicated otherwise "%" refers to wt-% (% by weight).

Within the meaning of the invention, the term "permeation enhancer" refers to a substance which increases the active agent permeability, e.g., by influencing the barrier properties of the epithelial layer such as, e.g., stratified squamous keratinized epithelium or non-keratinized epithelium . For example, penetration enhancers may influence the structure of the tight junctions or lipid vesicles in the non-keratinized epithelium. Suitable permeation enhancers according to the invention are C6 to C12 fatty acids and salts thereof and bile acids and salts thereof. In this context, the term "fatty acid" is understood to mean an aliphatic compound comprising at least one carboxylic acid group (COOH). The fatty acid may be saturated or unsaturated, linear or branched, and cyclic or acyclic. For example, the fatty acid may be caproic acid, heptanoic acid, caprylic acid, nonanoic acid, capric acid, undecanoic acid or lauric acid. The term "bile acid" as used herein, includes any steroid acids (and/or the carboxylate anion thereof), and salts thereof, found in the bile of an animal (e.g., a human), including, by way of non-limiting example, cholic acid, cholate, deoxycholic acid, deoxycholate, hyodeoxycholic acid, hyodeoxycholate, glycocholic acid, glycocholate, taurocholic acid, taurocholate, chenodeoxycholic acid, chenodeoxycholate, lithocholic acid, lithocholate, and the like.

Within the meaning of the invention, the term "salt" is used in the broadest sense and preferably is a pharmaceutically acceptable salt. For example, the term "salt" includes alkali salts of C6 to C12 fatty acids such as sodium caprate as well as alkali salts of bile acids such as sodium glycocholate.

Within the meaning of this invention, the term "polymer" refers to any substance consisting of so-called repeating units obtained by polymerizing one or more monomers, and includes homopolymers which consist of one type of monomer and copolymers which consist of two or more types of monomers. Polymers may be of any architecture such as linear polymers, star polymer, comb polymers, brush polymers, of any monomer arrangements in case of copolymers, e.g. alternating, statistical, block copolymers, or graft polymers. The minimum molecular weight varies depending on the polymer type and is known to the skilled person. Polymers may e.g. have a molecular weight above 2,000, preferably above 5,000 and more preferably above 10,000 g/mol. Correspondingly, compounds with a molecular weight below 2,000, preferably below 5,000 or more preferably below 10,000 g/mol are usually referred to as oligomers.

The transmucosal therapeutic systems according to the present invention can be characterized by certain parameters as measured in an *in vitro* permeation test.

The *in vitro* permeation test can be performed with human or animal mucosa or with an oral mucosal model (human organotypic oral epithelial tissue cultures), e.g., with phosphate buffer pH 7.4 as receptor medium (37 °C) with or without addition of a maximum of 20 vol-% organic solvent. Where not otherwise indicated, the *in vitro* permeation test is performed with human organotypic oral epithelial tissue cultures, and with phosphate buffer pH 7.4 (with 0.1 % (w/v%) sodium azide as antibacteriological agent) as receptor medium (37 °C). Human organotypic oral epithelial tissue cultures are commercially available under the brand name EpiOral^{™} (ORL-200), developed by MatTek. The tissue consists of normal, human-derived epithelial cells. The cells have been cultured to form multilayered, highly differentiated models of the human buccal (EpiOral) phenotypes. The EpiOral tissue model exhibits *in vivo-*like morphological and growth characteristics which are uniform and highly reproducible. Morphologically, the tissue model closely parallels native human tissue, thus providing a useful *in-vitro* means to assess *in-vivo* permeability of pharmaceutical formulations across buccal mucosa.

The amount of active permeated into the receptor medium is determined in regular intervals using an HPLC method with a UV photometric detector by taking a sample volume. The measured amount of active permeated relates to the amount permeated between the two last sampling points and not the total amount permeated so far.

Thus, within the meaning of this invention, the parameter "permeated amount" is provided in µg/cm² and relates to the amount of active permeated in a sample interval at certain elapsed time per area of release. E.g., in an *in vitro* permeation test as described above, wherein the amount of active permeated into the receptor medium has been e.g. measured at hours 1, 2, 3, (4,) 5 and optionally 8, the "permeated amount" of active can be given e.g. for the sample interval from hour 2 to hour 3 and corresponds to the measurement at hour 3.

The permeated amount can also be given as a "cumulative permeated amount", corresponding to the cumulated amount of active permeated at a certain point in time. E.g., in an *in vitro* permeation test as described above, wherein the amount of active permeated into the receptor medium has been e.g. measured at hours 1, 2, 3, (4,) 5 and optionally 8, the "cumulative permeated amount" of active at hour 3 corresponds to the sum of the permeated amounts from hour 0 to hour 1, hour 1 to hour 2, and hour 2 to hour 3.

A "mucosa permeation rate" for a certain sample interval at certain elapsed time, provided in µg/(cm² h), can be calculated from the permeated amount in said sample interval as measured by *in vitro* permeation test as described above in µg/cm², divided by the hours of said sample interval. E.g. the mucosa permeation rate in an *in vitro* permeation test as described above, wherein the amount of active permeated into the receptor medium has been e.g. measured at hours 1, 2, 3, (4,) 5 and optionally 8, the "mucosa permeation rate" at hour 3 is calculated as the permeated amount in the sample interval from hour 2 to hour 3 divided by 1 hour.

A "cumulative mucosa permeation rate" can be calculated from the respective cumulative permeated amount by dividing the cumulative permeated amount by the elapsed time. E.g. in an *in vitro* permeation test as described above, wherein the amount of active permeated into the receptor medium has been e.g. measured at hours 1, 2, 3, (4,) 5 and optionally 8, the "cumulative mucosa permeation rate" at hour 3 is calculated as the cumulative permeated amount for hour 3 (see above) divided by 3 hours.

Within the meaning of this invention, the above parameters permeated amount and mucosa permeation rate as well as cumulative permeated amount and cumulative mucosa permeation rate refer to mean values calculated from 3 *in vitro* permeation test experiments. Where not otherwise indicated, the standard deviation (SD) of these mean values refer to a corrected sample standard deviation, calculated using the formula: $SD = \sqrt{\frac{1}{n - 1} {\sum }_{i = 1}^{n} {\left({x}_{i}-\overline{x}\right)}^{2}}$ wherein n is the sample size, {*x*₁, *x*₂, ... *xₙ*} are the observed values and *x̅* is the mean value of the observed values.

Within the meaning of the invention, the term "total amount permeated" refers to the entire amount of active permeated within the period of application. In this context, "the period of application" is understood to mean the time starting with administering the transmucosal therapeutic system until end ending with complete dissolution of the active agent-containing layer.

Within the meaning of this invention, the term "administration" refers to the application of the dosage form, i.e. the transmucosal therapeutic system, to the oral mucosa of the patient, which is then maintained on the mucosa until the active agent-containing layer is dissolved.

The skin irritation potential of the permeation enhancers according to the invention can be predicted using an *in vitro* cell viability assay. The test consists of an exposure of an oral mucosal model (human organotypic oral epithelial tissue cultures, EpiOral^{™} manufactured by MatTek) to a test substance comprising the penetration enhancers, followed by a cell viability test in accordance with the MTT effective time-50 ET-50) protocol (MatTek). In the test, cell viability is measured by dehydrogenase conversion of MTT [(3-4,5-dimethyl thiazole 2-yl) 2,5-diphenyltetrazoliumbromide], present in cell mitochondria, into a blue formazan salt that is quantitatively measured by optical density measurement (providing the absorbance, also called optical density, as OD) after extraction from tissues. The reduction of the viability of tissues exposed to chemicals in comparison to negative controls (treated with artificial saliva) is used to predict the skin irritation potential. For each individual tissue treated with a test substance (TS), the positive control (PC) and the negative control (NC) the individual relative tissue viability is calculated according to the following formulas:$Relative viability TS \left(%\right) = \left[ODTS/Mean of ODNC\right] \times 100$$Relative viability NC \left(%\right) = \left[ODNC/mean of ODNC\right] \times 100$$Relative viability PC \left(%\right) = \left[ODPC/mean of ODNC\right] \times 100 .$

Within the meaning of this invention, the term "room temperature" refers to the unmodified temperature found indoors in the laboratory where the experiments are conducted and usually lies within 15 to 35 °C, or about 18 to 25 °C.

Within the meaning of this invention, the term "patient" refers to a subject who has presented a clinical manifestation of a particular symptom or symptoms suggesting the need for treatment, who is treated preventatively or prophylactically for a condition, or who has been diagnosed with a condition to be treated. The condition may be diabetes and/or obesity.

Within the meaning of this invention, the term "coating composition" refers to a composition comprising all components of the drug-containing layer in a solvent, which may be coated onto the backing layer or release liner to form the drug-containing layer upon drying.

Within the meaning of this invention, the term "dissolve" in the context of the preparation of the coating composition, e.g. dissolving components of the coating composition such as the active agent, refers to the process of obtaining a solution, which is clear and does not contain any particles, as visible to the naked eye.

Within the meaning of this invention, the term "solvent" refers to any liquid substance, such as water.

Within the meaning of this invention, and unless otherwise specified, the term "about" refers to an amount that is ± 10 % of the disclosed amount. In some embodiments, the term "about" refers to an amount that is ± 5 % of the disclosed amount. In some embodiments, the term "about" refers to an amount that is ± 2 % of the disclosed amount.

### BRIEF DESCRIPTION OF THE DRAWINGS

Fig. 1a depicts the cumulative permeated amount of semaglutide across reconstructed human epithelial tissue after application of solutions according to Pre-Examples 1a, 1c and 1d for hours 0 to 8.
Fig. 1b depicts the cumulative permeated amount of semaglutide across reconstructed human epithelial tissue after application of solutions according to Pre-Examples 1a, 1e and 1f for hours 0 to 8.
Fig. 1c depicts the cumulative permeated amount of semaglutide across reconstructed human epithelial tissue after application of solutions according to Pre-Examples 1a, 1d, 1f and 1g for hours 0 to 8.
Fig. 1d depicts the utilization of semaglutide across reconstructed human epithelial tissue after application of solutions according to Pre-Examples 1a, 1c and 1d at hour 5.
Fig. 1e depicts the utilization of semaglutide across reconstructed human epithelial tissue after application of solutions according to Pre-Examples 1a, 1e and 1f at hour 5.
Fig. 1f depicts the utilization of semaglutide across reconstructed human epithelial tissue after application of solutions according to Pre-Examples 1a, 1d, 1f and 1g at hour 5.
Fig. 2a depicts the cumulative permeated amount of semaglutide across reconstructed human epithelial tissue after application of a solution according to Pre-Example 2a as well as a transmucosal therapeutic system according to Example 2b for hours 0 to 5.
Fig. 2b depicts the cumulative permeated amount of semaglutide across reconstructed human epithelial tissue after application of transmucosal therapeutic systems according to Examples 2b to 2d for hours 0 to 5.
Fig. 2c depicts the utilization of semaglutide across reconstructed human epithelial tissue after application of a solution according to Pre-Example 2a as well as transmucosal therapeutic systems according to Examples 2b to 2d at hour 5.
Fig. 3 depicts the cumulative permeated amount of semaglutide across reconstructed human epithelial tissue after application of a transmucosal therapeutic system according to Reference Example 3 for hours 0 to 5.
Fig. 4a depicts the cumulative permeated amount of semaglutide across reconstructed human epithelial tissue after application of transmucosal therapeutic systems according to Examples 4a, 4b and Reference Example 3 for hours 0 to 5.
Fig. 4b depicts the utilization of semaglutide across reconstructed human epithelial tissue after application of transmucosal therapeutic systems according to Examples 4a, 4b and Reference Example 3 at hour 5.
Fig. 5a depicts the cumulative permeated amount of semaglutide across reconstructed human epithelial tissue after application of transmucosal therapeutic systems according to Examples 5a, 5b and 5c for hours 0 to 5.
Fig. 5b depicts the utilization of semaglutide across reconstructed human epithelial tissue after application of transmucosal therapeutic systems according to Examples 5a, 5b and 5c at hour 5.
Fig. 6a depicts the cumulative permeated amount of tirzepatide across reconstructed human epithelial tissue after application of a transmucosal therapeutic system according to Example 6a for hours 0 to 5.
Fig. 6b depicts the cumulative permeated amount of liraglutide across reconstructed human epithelial tissue after application of transmucosal therapeutic systems according to Example 6b and Reference Example 6c for hours 0 to 5.
Fig. 6c depicts the utilization of tirzepatide across reconstructed human epithelial tissue after application of a transmucosal therapeutic system according to Example 6a at hour 5.
Fig. 6d depicts the utilization of liraglutide across reconstructed human epithelial tissue after application of transmucosal therapeutic systems according to Example 6b and Reference Example 6c at hour 5.
Fig. 7 depicts the MTT cell viability of reconstructed human epithelial tissue after application of a test substance (TS) as compared to a positive control (PC) for minutes 0 to 120 as measured according to the skin irritation potential experiment 7.

### DETAILED DESCRIPTION

### STRUCTURE OF THE TRANSMUCOSAL THERAPEUTIC SYSTEM

The present invention is related to a transmucosal therapeutic system for the transmucosal administration of a GLP-1 receptor agonist comprising a mucoadhesive layer structure containing a GLP-1 receptor agonist.

The mucoadhesive layer structure may contain therapeutically effective amounts of the GLP-1 receptor agonist. In certain embodiments, the mucoadhesive layer structure comprises at least 0.1 mg, at least 0.2 mg, or at least 0.4 mg GLP-1 receptor agonist, or the mucoadhesive layer structure comprises less than or equal to 20 mg, less than or equal to 15 mg, or less than or equal to 10 mg GLP-1 receptor agonist, or wherein the mucoadhesive layer structure comprises from 0.1 mg to 20 mg, from 0.2 mg to 15 mg, or from 0.4 mg to 10 mg GLP-1 receptor agonist.

In accordance with the invention, the mucoadhesive layer structure comprises an active agent-containing layer comprising a GLP-1 receptor agonist, a combination of permeation enhancers, and a dissolvable film-forming agent. Thus, the transmucosal therapeutic system for the transmucosal administration of a GLP-1 receptor agonist comprises a mucoadhesive layer structure, said mucoadhesive layer structure comprising
A) an active agent-containing layer.

The transmucosal therapeutic system of the present invention attempts to achieve a particularly high active delivery in order to ensure that the drug amount delivered is sufficient for enabling a therapeutically effective dose. As outlined in detail in the introductory section, so-called open systems, wherein active delivery is achieved by a combination of direct active release from the transmucosal therapeutic system to the mucosa, and by an indirect active delivery *via* dissolution in the saliva, are particularly advantageous in terms of high active permeation rates.

Thus, in certain embodiments of the present invention, the transmucosal therapeutic system does not comprise a backing layer.

In certain embodiments, the mucoadhesive layer structure further comprises one or more further layers selected from:
B) a mucosa-contacting layer, and
C) a cosmetic layer.

Thus, in certain embodiments, the mucoadhesive layer structure according to the invention comprises an additional mucosa-contacting layer. In certain other embodiments, the mucoadhesive layer structure according to the invention does not comprise an additional mucosa-contacting layer. In such and other embodiments, the active agent-containing layer may be mucoadhesive. The additional mucosa-contacting layer, if present, is mucoadhesive and provides for (improved) adhesion between the mucoadhesive layer structure and the mucosa of the patient during administration. A mucosa-contacting layer is provided just below the active agent-containing layer, and thus, forms an adhesive layer between the mucosa and the active agent-containing layer during administration. In view of convenience of manufacture and restricting the overall patch size, the size of the active agent-containing layer and the size of the mucosa-contacting layer are preferably coextensive.

As indicated above, the mucoadhesive layer structure may also comprise a cosmetic layer. Alternatively, the mucoadhesive layer structure may not comprise a cosmetic layer. In contrast to the mucosa-contacting layer, a cosmetic layer is located on top of the active agent-containing layer and is not (necessarily) intended to contact the mucosa.

As a result, according to certain embodiments, the mucoadhesive layer structure may further comprise one or more further layers selected from:
B) a mucosa-contacting layer, and
C) a cosmetic layer,
wherein
the further layers adjoin the active agent-containing layer, and
the mucosa-contacting layer and the cosmetic layer, if both are present, adjoin the active agent-containing layer on opposite sides.

The cosmetic layer may provide for a decorative means such as coloring or imprinting, or may simply prevent the patient from touching the active agent-containing layer during administration of the transmucosal therapeutic system. For such a protective function, it is preferable that the cosmetic layer covers the active agent-containing layer completely. Thus, in certain embodiments, the mucoadhesive layer structure further comprises a cosmetic layer, and the size of the active agent-containing layer and the size of the cosmetic layer are coextensive, or the cosmetic layer is larger in size than and extends the surface area of the active agent-containing layer.

On the other hand, a cosmetic layer is not to be confused with a backing layer. It is not a function of a cosmetic layer and would indeed be undesirable if the cosmetic layer posed an obstacle for the active to be released into the saliva. Thus, a cosmetic layer dissolves fast enough not to hinder the active to be dissolved into the saliva, and in certain embodiments, the cosmetic layer dissolves in water, in artificial or natural saliva, or in any other aqueous medium at 37 °C and 150 rpm, in less than 3 minutes, less than 1 minute, or in less than 30 seconds.

An elegant solution for a transmucosal therapeutic system, in terms of ease of manufacture and also in terms of simplicity, is, however, when the active agent-containing layer is the transmucosal therapeutic system itself. In other words, a preferred transmucosal therapeutic system of the present invention does neither comprise a mucosa-contacting layer, nor a cosmetic layer. Thus, according to certain embodiments, the mucoadhesive layer structure simply consists of the active agent-containing layer. In such and other embodiments, the active agent-containing layer is mucoadhesive, as it is preferred that the active agent-containing layer is able to directly adhere to the mucosa,

According to certain embodiments, the transmucosal therapeutic system further comprises a mucoadhesive overlay or does not comprise a mucoadhesive overlay, and preferably does not comprise a mucoadhesive overlay. This mucoadhesive overlay is in particular larger than the active agent-containing mucoadhesive layer structure and is attached thereto for enhancing the adhesive properties of the overall transmucosal therapeutic system. The area of said mucoadhesive overlay adds to the overall size of the transmucosal therapeutic system but does not add to the area of release. The mucoadhesive overlay comprises a mucoadhesive polymer or a mucoadhesive polymer mixture selected from the group of hydroxyethyl cellulose and hydroxypropyl cellulose, which may be identical to or different from any dissolvable film-forming agent included in the active agent-containing layer.

As outlined also above, a transmucosal therapeutic system consists of one or more thin layers, thus, in certain embodiments, the transmucosal therapeutic system is in the form of a film. Such a film may have a circular, rectangular or square shape.

The film preferably has a certain degree of thickness, as otherwise it will be difficult to incorporate the required amount of active, and as very thin films are not easy to manufacture, in particular with respect to providing an even thickness. Thus, in certain embodiments, the transmucosal therapeutic system is in the form of a thin film having an area weight of at least 40 g/m², at least 80 g/m², or at least 100 g/m². On the other hand, very thick films will be perceived by the patient as a disturbing object in the oral cavity, and thus are disadvantageous in terms of patient compliance. Thus, in certain embodiments, the transmucosal therapeutic system is in the form of a thin film having an area weight of less than or equal to 500 g/m², less than or equal to 300 g/m², or less than or equal to 250 g/m². In particular, according to certain embodiments, the transmucosal therapeutic system is in the form of a thin film having an area weight of from 40 to 500 g/m², from 80 to 300 g/m², or from 80 to 250 g/m².

In an open system transmucosal therapeutic system comprising no backing layer, the active delivery is controlled by a combination of direct and indirect delivery as explained above, which is why in preferred embodiments, the area of release, i.e. the surface area of the active agent-containing layer, plays a minor role in the control of the effective dose. However, a certain minimum size is required in order to ensure that the patch does not detach prematurely from the mucosa, and also for being able to include a sufficient amount of active without having to use very thick films. On the other hand, if the area of release is too large, the transmucosal therapeutic system will be huge in size, uncomfortable to apply and to wear, leading to low patient compliance. Considering this, according to certain embodiments, the transmucosal therapeutic system has an area of release of at least 0.1 cm², at least 0.2 cm², or at least 0.5 cm², or has an area of release of less than or equal to 10 cm², less than or equal to 7 cm², or less than or equal to 5 cm², or has an area of release of from 0.1 to 10 cm², from 0.2 to 7 cm², or from 0.5 to 5 cm².

The transmucosal therapeutic system according to the invention is normally stored in a seam-sealed pouch without any further means of protection. However, the mucoadhesive layer structure may also be located on a detachable protective layer (release liner) from which it is removed immediately before application to the mucosa of the patient's oral cavity. Thus, the transmucosal therapeutic system may or may not further comprise a release liner. A transmucosal therapeutic system protected by a release liner is usually also stored in a seam-sealed pouch. The packaging may be child resistant and/or senior friendly.

### ACTIVE AGENT-CONTAINING LAYER

As outlined in more detail above, the transmucosal therapeutic system according to the present invention comprises a mucoadhesive layer structure comprising an active agent-containing layer. The active agent-containing layer comprises
1. a GLP-1 receptor agonist;
2. a combination of permeation enhancers; and
3. a dissolvable film-forming agent.

In a specific embodiment, the active agent-containing layer may comprise
1. a GLP-1 receptor agonist in an amount of from 2 wt-% to less than or equal to 10 wt-%;
2. a combination of permeation enhancers in an amount of from 12 wt-% to less than or equal to 36 wt-%; and
3. a dissolvable film-forming agent in an amount of from 40 wt-% to less than or equal to 60 wt-%.

Without wishing to be bound by theory, it is believed that a sufficient amount of active agent contained in the transmucosal therapeutic system is necessary to achieve certain advantageous features of the transmucosal therapeutic system according to the present invention, such as good *in vitro* permeation. The amount of GLP-1 receptor agonist contained in the transmucosal therapeutic system can be controlled two-way by adjusting content and/or the area weight of the active agent-containing layer.

The area weight of the active agent-containing layer is one of the factors decisive for the amount of active. A certain thickness is required in order to obtain a sufficient amount of active, and it is also difficult to coat very thin layers in particular with sufficient accuracy. On the other hand, thick layers may not only provoke an uncomfortable feeling in the oral cavity, but are also difficult to manufacture, and may result in the layer taking too long to dissolve for the desired release profile. On balance, according to certain embodiments, the active agent-containing layer has an area weight of at least 40 g/m², at least 80 g/m², or at least 100 g/m², or has an area weight of less than or equal to 500 g/m², less than or equal to 300 g/m², or less than or equal to 250 g/m², or has an area weight of from 50 to 500 g/m², from 100 to 300 g/m², or from 120 to 25 g/m².

In terms of the content, according to certain embodiments, the active agent-containing layer comprises at least 0.01 mg/cm², at least 0.05 mg/cm², at least 0.1 mg/cm², at least 0.2 mg/cm², or at least 0.4 mg/cm² GLP-1 receptor agonist, or the active agent-containing layer comprises less than or equal to 2.0 mg/cm², less than or equal to 1.2 mg/cm², or less than or equal to 1.0 mg/cm² GLP-1 receptor agonist, or the active agent-containing layer comprises from 0.1 to 2.0 mg/cm², from 0.2 to 1.2 mg/cm², or from 0.4 to 1.0 mg/cm² GLP-1 receptor agonist (per area of release).

The content of GLP-1 receptor agonist may be reduced, if an additional antidiabetic is administered concurrently with the GLP-1 receptor agonist. Thus, the active agent-containing layer may further comprise an additional antidiabetic, in particular basal insulin such as insulin degludec or insulin glargine.

According to certain embodiments, the active agent-containing layer is obtainable (and/or is obtained) by drying a coated coating composition comprising the GLP-1 receptor agonist, the combination of permeation enhancers, the dissolvable film-forming agent, and water. In terms of the amount of water, the active agent-containing layer may obtainable (and/or may be obtained) by drying a coated coating composition comprising less than 75 wt-%, less than 50 wt-%, or less than 30 wt-% water.

Considering the stability of the active agent-containing layer with respect to its composition, it is preferable that the active agent-containing layer does not comprise any volatile constituents, which bear the risk of evaporating and changing the composition upon storage. Thus, in certain embodiments, the active agent-containing layer comprises substantially no volatile solvent. A volatile solvent in this sense may be selected from the group consisting of C1 to C3 linear and branched alcohols, ethyl acetate, hexane, n-heptane, and any mixtures thereof. In view of the transmucosal therapeutic system being applied in the oral cavity, the volatile solvents include particularly those which should better not be digested such as methanol, ethyl acetate, hexane, n-heptane, and mixtures thereof. In particular, the active agent-containing layer comprises less than or equal to 5 wt-%, less than or equal to 3 wt-%, or less than or equal to 1 wt-% volatile solvent.

Further, according to certain embodiments, the active agent-containing layer (subsequent to drying) comprises substantially no water. In particular, according to certain embodiments, the active agent-containing layer comprises less than or equal to 12 wt-%, less than or equal to 8 wt-%, less than or equal to 5 wt-%, or less than or equal to 4 wt-% water.

### GLP-1 RECEPTOR AGONIST

In accordance with the invention, the mucoadhesive layer structure preferably contains a GLP-1 receptor agonist in a therapeutically effective amount. In particular, the active agent-containing layer of the mucoadhesive layer structure of the transmucosal therapeutic system according to the invention comprises at least one GLP-1 receptor agonist.

The GLP-1 receptor agonist used in the transmucosal therapeutic system according to the present invention is not particularly limited as long as it is suitable for transmucosal delivery. Accordingly, the GLP-1 receptor agonist can be selected from peptides or peptide conjugates exhibiting at least partial sequence identity, such as at least 20% sequence identity, at least 50% sequence identity, at least 75% sequence identity or at least 90% sequence identity, with endogenous GLP-1(7-37).

Surprisingly, it has been found that GLP-1 receptor agonists having a molecular weight of 1,000 g/mol or more, such as 3,000 g/mol or more, can be delivered transmucosally in sufficient amounts when using a specific combination of permeation enhancers as described in detail further below into the active agent-containing layer of the transmucosal therapeutic system according to the invention. Thus, according to certain embodiments, the GLP-1 receptor agonist is selected from peptides or peptide conjugates having a molecular weight of 1,000 g/mol or more, 3,000 g/mol or more, 3,500 g/mol or more, or 3,750 g/mol or more, or the GLP-1 receptor agonist is selected from peptides or peptide conjugates having a molecular weight of 100,000 g/mol or less, 20,000 g/mol or less, 10,000 g/mol or less, or 5,000 g/mol or less. In particular, the GLP-1 receptor agonist may be selected from peptides or peptide conjugates having a molecular weight of 1,000 g/mol or more and 100,000 g/mol or less, 3,000 g/mol or more and 20,000 g/mol or less, 3,500 g/mol or more and 10,000 g/mol or less, or 3,750 g/mol or more and 5,000 g/mol or less.

In certain embodiments, the GLP-1 receptor agonist is selected from the group consisting of albiglutide, beinaglutide, cotadutide, dulaglutide, efinopegdutide, efocipegtrutide, efpeglenatide, exenatide, liraglutide, lixisenatide, pemvidutide, retatrutide, semaglutide, survodutide, taspoglutide, and tirzepatide. In certain specific embodiments, the GLP-1 receptor agonist is selected from the group consisting of liraglutide, semaglutide and tirzepatide. Thus, according to certain specific embodiments, the active agent-containing layer comprises
1. liraglutide, semaglutide or tirzepatide;
2. a combination of permeation enhancers; and
3. a dissolvable film-forming agent.

In certain more specific embodiments, the GLP-1 receptor agonist is semaglutide. Thus, according to contain specific embodiments, the active agent-containing layer comprises
1. semaglutide;
2. a combination of permeation enhancers; and
3. a dissolvable film-forming agent.

According to certain embodiments, the amount of the GLP-1 receptor agonist is at least 0.01 wt-%, at least 0.1 wt-%, at least 0.5 wt-%, at least 1 wt-%, or at least 2 wt-% of the active agent-containing layer. Also, according to certain embodiments, the amount of the GLP-1 receptor agonist is less than or equal to 25 wt-%, less than or equal to 20 wt-%, or less than or equal to 10 wt-% of the active agent-containing layer. In particular, according to certain embodiments, the amount of GLP-1 receptor agonist is from 0.5 to less than or equal to 25 wt-%, from 1 to less than or equal to 20 wt-%, or from 2 to less than or equal to 10 wt-% of the active agent-containing layer.

As outlined above, the transmucosal therapeutic system of the invention provides for a high active ingredient utilization and sufficient release of the GLP-1 receptor agonist within a period of application of less than 5 hours or less than 3 hours. Typically, a therapeutically effective amount of the GLP-1 receptor agonist is released from the transmucosal therapeutic system within a period of application of from about 30 minutes to about 3 hours, or from about 45 minutes to about 2 hours.

### PERMEATION ENHANCERS

As outlined above, the active agent-containing layer of the mucoadhesive layer structure of the transmucosal therapeutic system according to the invention comprises a combination of permeation enhancers comprising
i) a first permeation enhancer selected from the group consisting of C6 to C12 fatty acids and salts thereof; and
ii) a second permeation enhancer selected from the group consisting of bile acids and salts thereof.

In certain embodiments, the first permeation enhancer is selected from the group consisting of caproic acid, heptanoic acid, caprylic acid, nonanoic acid, capric acid, undecanoic acid, lauric acid and salts thereof. In certain specific embodiments, the first permeation enhancer is selected from the group consisting of caproic acid, caprylic acid, capric acid, lauric acid and salts thereof. In particular, according to certain specific embodiments, the first permeation enhancer is a salt, such as an alkali salt, of a fatty acid selected from the group consisting of caproic acid, caprylic acid, capric acid and lauric acid, such as a sodium salt or potassium salt of a fatty acid selected from the group consisting of caproic acid, caprylic acid, capric acid or lauric acid.

Thus, according to certain specific embodiments, the first permeation enhancer is selected from the group consisting of sodium caproate, sodium caprylate, sodium caprate, sodium laurate, potassium caproate, potassium caprylate, potassium caprate, and potassium laurate. In certain more specific embodiments, the first permeation enhancer is selected from the group consisting of sodium caprate and potassium caprate.

In particular, according to further specific embodiments, the first permeation enhancer is sodium caprate.

According to certain embodiments, if the first permeation enhancer is sodium caprate, the second permeation enhancer is not sodium chenodeoxycholate. In particular, if the first permeation enhancer is sodium caprate, the second permeation enhancer may be selected from the group consisting of sodium cholate, sodium taurocholate, sodium glycocholate, sodium deoxycholate, sodium taurodeoxycholate, sodium glycodeoxycholate, sodium ursodeoxycholate, sodium taurochenodeoxycholate, sodium glycochenodeoxycholate, sodium cholylsarcosine and sodium N-methyl taurocholate.

According to certain other embodiments, if the first permeation enhancer is sodium caprate, the second permeation enhancer is not sodium deoxycholate. In particular, if the first permeation enhancer is sodium caprate, the second permeation enhancer may be selected from the group consisting of sodium cholate, sodium taurocholate, sodium glycocholate, sodium chenodeoxycholate, sodium taurodeoxycholate, sodium glycodeoxycholate, sodium ursodeoxycholate, sodium taurochenodeoxycholate, sodium glycochenodeoxycholate, sodium cholylsarcosine and sodium N-methyl taurocholate.

In these or other embodiments, the second permeation enhancer is selected from the group consisting of cholic acid, taurocholic acid, glycocholic acid, deoxycholic acid, taurodeoxycholic acid, glycodeoxycholic acid, chenodeoxycholic acid, taurochenodeoxycholic acid, glycochenodeoxycholic acid, and salts thereof. In particular, according to certain specific embodiments, the second permeation enhancer is a salt, such as an alkali salt, of a bile acid selected from the group consisting of cholic acid, taurocholic acid, glycocholic acid, deoxycholic acid, taurodeoxycholic acid, glycodeoxycholic acid, chenodeoxycholic acid, taurochenodeoxycholic acid, and glycochenodeoxycholic acid, such as a sodium salt of a bile acid selected from the group consisting of cholic acid, taurocholic acid, glycocholic acid, deoxycholic acid, taurodeoxycholic acid, glycodeoxycholic acid, chenodeoxycholic acid, taurochenodeoxycholic acid, and glycochenodeoxycholic acid.

Thus, according to certain specific embodiments, the second permeation enhancer is selected from the group consisting of sodium cholate, sodium taurocholate, sodium glycocholate, sodium deoxycholate, sodium taurodeoxycholate, sodium glycodeoxycholate, sodium ursodeoxycholate, sodium chenodeoxycholate, sodium taurochenodeoxycholate, sodium glycochenodeoxycholate, sodium cholylsarcosine and sodium N-methyl taurocholate. In certain more specific embodiments, the second permeation enhancer is selected from the group consisting of sodium glycocholate and sodium taurotaurodeoxycholate.

In particular, according to further specific embodiments, the second permeation enhancer is sodium glycocholate.

According to certain specific embodiments,
i) the first permeation enhancer is a salt of a fatty acid selected from the group consisting of caproic acid, caprylic acid, capric acid and lauric acid; and
ii) the second permeation enhancer is a salt of a bile acid selected from the group consisting of cholic acid, taurocholic acid, glycocholic acid, deoxycholic acid, taurodeoxycholic acid, glycodeoxycholic acid, ursodeoxycholic acid, chenodeoxycholic acid, taurochenodeoxycholic acid, glycochenodeoxycholic acid, sarcocholic acid and N-methyl taurocholic acid,
or
i) the first permeation enhancer is selected from the group consisting of sodium caproate, sodium caprylate, sodium caprate, sodium laurate, potassium caproate, potassium caprylate, potassium caprate, and potassium laurate; and
ii) the second permeation enhancer is selected from the group consisting of sodium cholate, sodium taurocholate, sodium glycocholate, sodium deoxycholate, sodium taurodeoxycholate, sodium glycodeoxycholate, sodium ursodeoxycholate, sodium chenodeoxycholate, sodium taurochenodeoxycholate, sodium glycochenodeoxycholate, sodium cholylsarcosine and sodium N-methyl taurocholate,
or
i) the first permeation enhancer is selected from the group consisting of sodium caprate and potassium caprate; and
ii) the second permeation enhancer is selected from the group consisting of sodium glycocholate and sodium taurodeoxycholate.

According to certain particular embodiments,
i) the first permeation enhancer is sodium caprate; and
ii) the second permeation enhancer sodium glycocholate. Thus, according to certain particular embodiments, the active agent-containing layer comprises
   1. a GLP-1 receptor agonist;
   2. a combination of permeation enhancers comprising a first and a second permeation enhancer, wherein the first permeation enhancer is sodium caprate and the second permeation enhancer is sodium glycocholate; and
   3. a dissolvable film-forming agent.

The permeation enhancers as defined herein increase the amount of GLP-1 receptor agonist released from the transmucosal therapeutic system according to the invention to the systemic circulation. Without wishing to be bound by theory, it is believed that the permeation enhancers may *inter alia* affect the structure or the fluidization of the transcellular membrane to increase the permeability, and may also act as a solubiliser for GLP-1 receptor agonist.

Advantageously, the combination of the first permeation enhancer and the second permeation enhancer achieves a synergistic effect, resulting in an excellent and improved active delivery across the oral mucosa, which exceeds what would have been expected from the increase in active release obtained for each of the penetration enhancers independently. In this context, the weight ratio of first permeation enhancer to second permeation enhancer in the transmucosal therapeutic system according to the invention is adjusted accordingly in order to release sufficient amount of the GLP-1 receptor agonist within a reasonable period of application.

In certain embodiments, the weight ratio of first permeation enhancer to second permeation enhancer is at least 1:2, or at least 1:1. Also, in certain embodiments, the weight ratio of first permeation enhancer to second permeation enhancer is 10:1, or less or 6:1 or less, such as 3:1 or less, or 2:1 or less. In particular, according to certain embodiments, the weight ratio of first permeation enhancer to second permeation enhancer is from 1:2 to 10:1, or from 1:1 to 6:1. Moreover, according to certain specific embodiments, the weight ratio of first permeation enhancer to second permeation enhancer is about 1:1.

In addition to adjusting the ratio of first permeation enhancer to second permeation enhancer, the release of the GLP-1 receptor agonist from the transmucosal therapeutic system according to the invention further depends on the weight ratio of first permeation enhancer or second permeation enhancer, respectively, to GLP-1 receptor agonist. Thus, according to certain embodiments, the weight ratio of first or second permeation enhancer to GLP-1 receptor agonist is at least 0.3:1, at least 0.5:1, at least 1:1, or at least 2:1.

In certain embodiments, the weight ratio of first permeation enhancer to GLP-1 receptor agonist is at least 0.3:1, at least 1:1, at least 2:1 or at least 3:1. Also, according to certain embodiments, the weight ratio of first permeation enhancer to GLP-1 receptor agonist is 100:1 or less, 70:1 or less, 50:1 or less, or 40:1 or less, such as 30:1 or less, 10:1 or less, or 5:1 or less. In particular, according to certain embodiments, the weight ratio of first permeation enhancer to GLP-1 receptor agonist is from 0.3:1 to 100:1, from 1:1 to 70:1, from 2:1 to 50:1, or from 3:1 to 40:1. Moreover, according to certain specific embodiments, the weight ratio of first permeation enhancer to GLP-1 receptor agonist is from 2:1 to 10:1, or from 3:1 to 5:1.

In these or other embodiments, the weight ratio of second permeation enhancer to GLP-1 receptor agonist is at least 1.5:1, at least 3:1, at least 10:1, or at least 20:1. Also, in certain embodiments, the weight ratio of second permeation enhancer to GLP-1 receptor agonist is 100:1 or less, 70:1 or less, 50:1 or less, or 40:1 or less, such as 30:1 or less, 10:1 or less, or 5:1 or less. In particular, according to certain embodiments, the weight ratio of second permeation enhancer to GLP-1 receptor agonist is from 1.5:1 to 100:1, from 3:1 to 70:1, from 10:1 to 50:1, or from 20:1 to 40:1. Moreover, according to certain specific embodiments, the weight ratio of second permeation enhancer to GLP-1 receptor agonist is from 2:1 to 10:1, or from 3:1 to 5:1.

In certain particular embodiments, the weight ratios of first permeation enhancer to GLP-1 agonist and of second permeation enhancer to GLP-1 agonist are the same. In particular, according to certain particular embodiments, the weight ratios of first permeation enhancer to GLP-1 agonist and of second permeation enhancer to GLP-1 agonist are from 2:1 to 10:1, or from 3:1 to 5:1.

Accordingly, the weight ratio of first permeation enhancer to second permeation enhancer to GLP-1 receptor agonist is at least 2:2:1, or 10:10:1 or less. In certain particular embodiments, the weight ratio of first permeation enhancer to second permeation enhancer to GLP-1 receptor agonist is about 3:3:1, or about 4:4:1, or about 5:5:1.

As outlined also above and without wishing to be bound by theory, it is believed that increasing the amount of first and/or second permeation enhancer (and thus enlarging the weight ratio of first permeation enhancer or second permeation enhancer, respectively, to GLP-1 receptor agonist), also increases the amount of GLP-1 receptor agonist released from the transmucosal therapeutic system according to the invention. In other words, a certain amount of enhancer is needed in order to ensure a sufficient degree of increase in active delivery. On the other hand, if the amount of first and/or second permeation enhancer is too high, the composition will be difficult to formulate, in particular since a larger amount of enhancer will result in increased size or volume of the medicament. In addition, a large amount of enhancer might lead to bade taste which is difficult to mask as well as to potential irritating sensations at the mucosa or otherwise in the oral cavity. Thus, the amount of first permeation enhancer and/or second permeation enhancer is preferably adjusted in the transmucosal therapeutic system according to the invention such that during and following the period of application (of, e.g., from about 10 minutes to about 5 hours, from about 30 minutes to about 3 hours, from about 45 minutes to about 2 hours, or from about 50 minutes to about 90 minutes) no or only mild irritating sensations occur at the mucosa or otherwise in the oral cavity. The skin irritation potential of the enhancers can be predicted by using a MTT cell viability assay as described in Example 7.

According to certain embodiments, the amount of the first permeation enhancer is from 3 wt-% to less than or equal to 30 wt-%, from 50 wt-% to less than or equal to 21 wt-%, or from 6 wt-% to less than or equal to 18 wt-% of the active agent-containing layer. In these or other embodiments, the amount of the second permeation enhancer is from 3 wt-% to less than or equal to 30 wt-%, from 50 wt-% to less than or equal to 21 wt-%, or from 6 wt-% to less than or equal to 18 wt-% of the active agent-containing layer. In certain particular embodiments, the amount of the first permeation enhancer and of the second permeation enhancer is the same. In particular, according to certain particular embodiments, each the amount of the first permeation enhancer and of the second permeation enhancer is from 6 wt-% to less than or equal to 18 wt-%, such as from 9 wt-% to less than or equal to 15 wt-%.

Further, according to certain embodiments, the amount of the combination of permeation enhancers is from 6 wt-% to less than or equal to 60 wt-%, from 10 wt-% to less than or equal to 42 wt-%, or from 12 wt-% to less than or equal to 36 wt-% of the active agent-containing layer, such as from 18 wt-% to less than or equal to 30 wt.-%.

### DISSOLVABLE FILM-FORMING AGENT

As outlined above, the active agent-containing layer of the mucoadhesive layer structure of the transmucosal therapeutic system according to the invention comprises a dissolvable film-forming agent.

This dissolvable film-forming agent provides for sufficient cohesion of the active agent-containing layer as long as it is kept in dry state. According to certain embodiments, the dissolvable film-forming agent may also provide for sufficient adhesion to the mucosa once wet, i.e. when having been brought in contact with the mucosa. In such embodiments, but also in general, dissolvable film-forming agent may be selected from mucoadhesive polymers.

The film-forming agent is not particularly limited as long as it is "dissolvable".

The film-forming agent is the primary control over the dissolution behavior of the active agent-containing layer. This is why the film-forming agent is "dissolvable". This means in certain specific embodiments that the dissolvable film-forming agent, if casted into a film having an area weight of from 100 to 250 g/m², or of 200 g/m², dissolves in water, in artificial or natural saliva, or in any other aqueous medium, at 37 °C and 150 rpm, in less than 5 hours, less than 3 hours, less than 2 hours, or less than 1.5 hours or 90 minutes. The dissolvable film-forming agent, if casted into a film having an area weight of from 100 to 250 g/m², or of 200 g/m², may also dissolve in water, in artificial or natural saliva, or in any other aqueous medium, at 37 °C and 150 rpm, in more than 10 minutes, more than 30 minutes, more than 45 minutes, or more than 50 minutes. In particular, the dissolvable film-forming agent, if casted into a film having an area weight of from 100 to 150 g/m², or of 200 g/m², may dissolve in more than 10 minutes and less than 5 hours, more than 30 minutes and less than 3 hours, more than 45 minutes and less than 2 hours, or more than 50 minutes and less than 1.5 hours.

Film-forming agents which are suitable as the dissolvable film-forming agent in accordance with the invention are, e.g., selected from the group consisting of polymers such as polyvinylpyrrolidone (commercially available as Kollidon^{®} 30F from BASF), methyl cellulose (commercially available as Methocel^{®} from Colorcon), ethyl cellulose (commercially available as Ethocel^{®} from Colorcon), hydroxyethyl cellulose (commercially available as Natrosol^{®} 250 L from Ashland Industries), hydroxypropyl cellulose (commercially available as Klucel^{®} from Ashland Industries), hydroxypropyl methyl cellulose (also known as hypromellose, commercially available from various suppliers, e.g., as Pharmacoat^{®} from Shin-Etsu, Methocel^{™} from Dow Chemical, or as Benecel^{™} from Ashland Industries), carboxymethyl cellulose sodium (uncrosslinked sodium salt of carboxymethyl cellulose also referred to as CMC or carmellose, commercially available as Blanose^{®} from Ashland Industries), polyethylene glycol- polyvinyl acetate- and polyvinylcaprolactame-based graft copolymers (commercially available as Soluplus^{®} from BASF), polyvinyl alcohol (commercially available as Mowiol^{®} 4-88 from Kuraray), polyvinyl alcohol-polyethylene glycol copolymers (commercially available as Kollicoat^{®} IR from BASF), polyvinylpyrrolidone-polyvinylacetate copolymers (also referred to as copovidones and commercially available e.g. as Kollidon^{®} VA64 from BASF), polyethylene oxides, polyethylene glycols, methacrylic acid - methyl methacrylate copolymers (commercially available as Eudragit^{®} L100, Eudragit^{®} L12,5, Eudragit^{®} S100 and Eudragit^{®} S12,5 from Evonik), and methacrylic acid - ethyl methacrylate copolymers (commercially available as Eudragit^{®} L100-55 and Eudragit^{®} L30D55 from Evonik), and natural film-forming agents such as shellac, pectin, gelatine, alginate, pullulan and starch derivatives, and any mixtures thereof.

The dissolvable film-forming agent should be able not only to provide sufficient cohesion to the active agent-containing layer, but preferably provides a film that is not tacky in dry state so that the patient is able to touch and manipulate the active agent-containing layer, e.g. apply it to the oral mucosa, without the same adhering to the fingers. In addition, since the dissolvable film-forming agent is the primary control over the dissolution behavior of the active agent-containing layer which needs to be neither too fast nor too slow, the dissolvable film-forming agent is preferably soluble, dispersible or otherwise disintegrable in aqueous media, specifically in saliva, or, simplified, in water. Thus, selecting the film-forming agent is not a simple task.

In certain embodiments, the dissolvable film-forming agent is selected from the group consisting of polymers such as polyvinylpyrrolidone, methyl cellulose, hydroxyethyl cellulose, hydroxypropyl cellulose, hydroxypropyl methyl cellulose, carboxymethyl cellulose sodium, polyethylene glycol- polyvinyl acetate- and polyvinylcaprolactame-based graft copolymers, polyvinyl alcohol, polyvinyl alcohol-polyethylene glycol copolymers, polyvinylpyrrolidone-polyvinylacetate copolymers, polyethylene oxides, polyethylene glycols, and any mixtures thereof. In particular, according to certain embodiments, the dissolvable film-forming agent is selected from the group consisting of polymers such as methyl cellulose, hydroxyethyl cellulose, hydroxypropyl cellulose, hydroxypropyl methyl cellulose, carboxymethyl cellulose sodium, and any mixtures thereof.

According to certain specific embodiments, the dissolvable film-forming agent comprises a hydroxypropyl methyl cellulose or a mixture of hydroxypropyl methyl celluloses. In particular, according to certain specific embodiments, the dissolvable film-forming agent comprises a mixture of at least two hydroxypropyl methyl celluloses, such as a mixture of a first hydroxypropyl methyl cellulose, a second hydroxypropyl methyl cellulose, and optionally a third and further hydroxypropyl methyl celluloses.

The first, second, third and any further hydroxypropyl methyl celluloses used as dissolvable film-forming agent in the active agent-containing layer of the mucoadhesive layer structure of the transmucosal therapeutic system according to the invention may differ from each other by viscosity, as preferably determined for 2% (w/v) aqueous solutions (measured at 20°C), in particular by using Ubbelohde (capillary) viscometers for products with viscosity less than 600 mPa s or Brookfield (rotational) viscometers for products with viscosity greater than or equal to 600 mPa s . Thus, according to certain embodiments, the dissolvable film-forming agent comprises a hydroxypropyl methyl cellulose having a viscosity of 10 mPa s or less, 5 mPa s or less, or 3 mPa s or less, and/or the dissolvable film-forming agent comprises a hydroxypropyl methyl cellulose having a viscosity of at least 10 mPa s, at least 50 mPa s, or at least 80 mPa s.

In certain embodiments, the dissolvable film-forming agent comprises a first hydroxypropyl methyl cellulose having a viscosity of from 1.5 mPa s to 5 mPa s or less, from 2 mPa s to 4 mPa s or less, or from 2.4 mPa s to 3.6 mPa s or less, and a second hydroxypropyl cellulose having a viscosity of from 10 mPa s to 20 mPa s or less, from 18.5 mPa s to 11.5 mPa s or less, or from 12 mPa s to 18 mPa s or less.

In certain other embodiments, the dissolvable film-forming agent comprises a mixture of a first hydroxypropyl methyl cellulose having a viscosity of from 1.5 mPa s to 5 mPa s or less, from 2 mPa s to 4 mPa s or less, or from 2.4 mPa s to 3.6 mPa s or less, such as about 3 mPa s, and a second hydroxypropyl cellulose having a viscosity of from 10 mPa s to 200 mPa s or less, from 50 mPa s to 150 mPa s or less, or from 80 mPa s to 120 mPa s or less, such as about 100 mPa s.

In any of the above embodiments, the dissolvable film-forming agent may further comprise a third hydroxypropyl methyl cellulose having a viscosity of from 10,000 mPa s to 250,000 mPa s, from 50,000 mPa s to 180,000 mPa s, or from 75,000 mPa s to 140,000 mPa s, such as about 100,000 mPa s. Thus, according to certain embodiments,

Further, according to certain embodiments, the hydroxypropyl methyl cellulose, in particular the first, second, third or any further hydroxypropyl methyl celluloses, used as dissolvable film-forming agent in the active agent-containing layer of the mucoadhesive layer structure of the transmucosal therapeutic system has a methoxyl content of from 10 % to less than or equal to 40 %, such as a methoxyl content of from 16% to less than or equal to 28%, or from 19% or 20% to less than or equal to 24%, or a methoxyl content of from 24% to less than or equal to 34%, or from 28% to less than or equal to 30%, and/or a hydroxypropoxyl content of from 2 % to less than or equal to 17 %, from 5 % to less than or equal to 14 %, or from 7 % to less than or equal to 12 %.

Hydroxypropyl methyl celluloses are commercially available from Shin-Etsu under the brand name Pharmacoat^{®}, e.g., with the following properties:
Pharmacoat^{®} 603
Viscosity: 2.4-3.6 mPa s, Methoxyl content: 28-30%, Hydroxypropyl content: 7-12%
Pharmacoat^{®} 615
Viscosity: 12.0-18.0 mPa s, Methoxyl content: 28-30%, Hydroxypropyl content: 7-12%

Hydroxypropyl methyl celluloses are also commercially available from Dow Chemical under the brand name Methocel^{™}, e.g., with the following properties:
Methocel^{™} K100LV
Viscosity: 80-120 mPa s, Methoxyl content: 19-24%, Hydroxypropyl content: 7-12%

Hydroxypropyl methyl celluloses are also commercially available from Ashland under the brand name Benecel^{™}, e.g., with the following properties:
Benecel^{™} K100M
Viscosity: 75,000-140,000 mPa s, Methoxyl content: 20-24%, Hydroxypropyl content: 7-12%

In order to be able to provide sufficient cohesion to the active agent-containing layer, a certain amount of the dissolvable film-forming agent should be included. Thus, according to certain embodiments, the amount of the dissolvable film-forming agent is at least 25 wt-%, at least 30 wt-% or at least 40 wt-%, or the amount of the dissolvable film-forming agent is less than or equal to 75 wt-%, less than or equal to 70 wt-% or less than or equal to 60 wt-%, or the amount of the dissolvable film-forming agent ranges from 25 to 75 wt-%, from 30 to 70 wt-%, or from 40 to 60 wt-% of the active agent-containing layer.

Such a film-forming agent may be present as the dissolvable film-forming agent in the active agent-containing layer, but may also be contained in an optional overlay.

### FURTHER ADDITIVES

The active agent-containing layer of the transmucosal therapeutic system according to the invention may comprise further excipients or additives selected from the group consisting of taste masking agents, sweeteners, flavoring agents, colorants, permeation enhancers, solubilizers, plasticizers, humectants, disintegrants, emulsifiers, antioxidants, stabilizers, buffer reagents, further permeation enhancers and further film-forming agents.

Such additives may be present in the active agent-containing layer in an amount of from 0.001 to 15 wt-% of the active agent-containing layer per additive. Hereinafter, where a range for an amount of a specific additive is given, such a range refers to the amount per individual additive.

It should be noted that in pharmaceutical formulations, the formulation components are categorized according to their physicochemical and physiological properties, and in accordance with their function. This means in particular that a substance or a compound falling into one category is not excluded from falling into another category of formulation component. The skilled person is able to determine based on his general knowledge in which category or categories of formulation component a certain substance or compound belongs to. In the following, details on the excipients and additives are provided which are, however, not to be understood as being exclusive. Other substances not explicitly listed in the present description may be as well used in accordance with the present invention, and substances and/or compounds explicitly listed for one category of formulation component are not excluded from being used as another formulation component in the sense of the present invention.

In view of the potentially unpleasant olfactory-gustatory sensation caused by the penetration enhancers used in the active agent-containing layer, substances that are able to mask or modify the same are preferred. Thus, according to certain embodiments, the active agent-containing layer comprises one or more excipients selected from the group consisting of taste masking agents, sweeteners, and flavoring agents.

In certain embodiments, the active agent-containing layer comprises one or more natural or artificial taste masking agents (such as ion-exchange agents) selected from the group consisting of flavor suppressing agents, bitter masking agents, sour masking agents and salty masking agents. In such embodiment, i.e. wherein the active agent containing layer comprises one or more natural or artificial taste masking agents, the amount of the taste masking agent at least 0.6 wt-%, at least 1.2 wt-% or at least 1.5 wt-%, or in an amount of less than or equal to 6.0 wt-%, less than or equal to 4.0 wt-% or less than or equal to 3.0 wt-%, or in an amount of from 0.6 to 6.0 wt-%, from 1.2 to 4.0 wt-%, or from 1.5 to 3.0 wt-% each.

In certain embodiments, the active agent-containing layer comprises one or more natural or artificial sweeteners selected from the group consisting of saccharose, glucose, fructose, sorbitol, mannitol, isomalt, maltitol, lactitol, xylitol, erythritol, sucralose, acesulfame potassium, aspartame, cyclamate, neohesperidine, neotame, steviol glycosides, thaumatin and saccharin sodium. In such embodiment, i.e. wherein the active agent-containing layer comprises one or more natural or artificial sweeteners, the amount of the sweetener is at least 0.05 wt-%, at least 0.1 wt-% or at least 0.15 wt-%, or in an amount of less than or equal to 2.0 wt-%, less than or equal to 1.5 wt-% or less than or equal to 1.0 wt-%, or in an amount of from 0.05 to 2.0 wt-%, from 0.1 to 1.5 wt-%, or from 0.15 to 1.0 wt-% each.

In certain embodiments, the active agent-containing layer comprises one or more natural or artificial flavoring agents selected from the group consisting of vanillin, methyl salicylate, menthol, manzanate, diacetyl, acetylpropionyl, acetoin, isoamyl acetate, benzaldehyde, cinnamaldehyde, ethyl propionate, methyl anthranilate, limonene, ethyl decadienoate, allyl hexanoate, ethyl maltol, 2,4-dithiapentane, ethylvanillin and eucalyptol as well as flavoring compositions such as peppermint flavor. In such embodiment, i.e. wherein the active agent-containing layer comprises one or more flavoring agents, the amount of the flavoring agent is at least 0.3 wt-%, at least 0.6 wt-% or at least 0.8 wt-%, or in an amount of less than or equal to 12 wt-%, less than or equal to 8 wt-%, or less than or equal to 6 wt-%, or in an amount of from 0.3 to 12 wt-%, from 0.6 to 8 wt-%, or from 0.8 to 6 wt-% each, and/or is from 0.3 to 20 wt-%, from 0.6 to 12 wt-%, or from 0.8 to 8 wt-% in total.

In certain embodiments, the active agent-containing layer comprises one or more colorants. Any colorant suitable for use in pharmaceutical / food applications can be included, in particular those admitted for use by the US FDA or by the European Agencies EFSA / EMA. Such colorants may be e.g. selected from the group consisting of titanium dioxide, brilliant blue FCF, indigo carmine, fast green FCF, erythrosine, allura red AC, tartrazine and sunset yellow FCF, curcumin, riboflavin, rivoflavin-5'-phosphate, quinoline yellow, orange yellow S, cochineal, carminic acid, azorubine, carmoisine, amaranth, ponceau 4R, cochineal red A, patent blue V, indigotine, chlorophylls, chlorophyllins, copper complexes of chlorophyll and chlorophyllins, green S, plain caramel, caustic sulphite caramel, ammonia caramel, sulphite ammonia caramel, brilliant black BN, black PN, vegetable carbon, brown HT, carotenes, annatto, bixin, norbixin, paprika extract, capsanthian, capsorubin, lycopene, beta-apo-8'-carotenal, lutein, canthaxanthin, beetroot red, betanin, anthocyanins, calcium carbonate, iron oxides and hydroxides, aluminium, silver, gold and litholrubine BK.

The active agent-containing layer may comprise one or more further film-forming agents in addition to those disclosed for the dissolvable film-forming agent above. Such a further film-forming agent is different from those disclosed previously for the dissolvable film-forming agent. The one or more further film-forming agents may be comprised in the active agent-containing layer in an amount of at least 0.5 wt-%, at least 2 wt-% or at least 3 wt-%, or in an amount of less than or equal to 20 wt-%, less than or equal to 15 wt-%, or less than or equal to 12 wt-%, or in an amount of from 0.5 to 20 wt-%, from 2 to 15 wt-%, or from 3 to 12 wt-% each, and/or in an amount from 0.5 to 30 wt-%, from 2 to 20 wt-%, or from 3 to 15 wt-% in total.

The active agent-containing layer may further comprise one or more solubilizers. Suitable solubilizers may be, e.g., selected from the group consisting of ethoxylated sorbitan esterified with fatty acids such as polyoxyethylene sorbitan monolaurate, polyoxyethylene sorbitan monopalmitate, polyoxyethylene sorbitan monostearate and polyoxyethylene sorbitan monooleate (commercially available as Tween 80 or Polysorbate 80), safflower oleosomes, propanediol and polyethoxylated castor oil.

The active agent-containing layer may further comprise one or more emulsifiers. Suitable emulsifiers may be, e.g., selected from the group consisting of soy lecithin, sodium phosphates, mono- and diglycerides of fatty acids, sodium stearoyl lactylate, diacetyl tartaric acid esters of mono- and diglycerides, and polyethoxylated hydrogenated castor oil (commercially available as Cremophor RH 40 from BASF).

The active agent-containing layer may further comprise one or more plasticizers. Suitable plasticizers may be selected from the group consisting of mono-, di-, oligo- and polysaccharides and derivatives such as sorbitol (commercially available as Sorbidex^{™} from Cargill), polyethylene glycol, triacetin, triethyl citrate, propylene glycol, glycerol and medium chain triglycerides.

### RELEASE CHARACTERISTICS

The transmucosal therapeutic systems in accordance with the invention are designed for transmucosally (such as oromucosally) administering a certain amount of GLP-1 receptor agonist to the systemic circulation.

An administration of the inventive transmucosal therapeutic system in general and preferably consists of applying the mucoadhesive layer structure (after removal of an eventually present release liner) to the mucosa of the oral cavity of a human patient and maintaining the same on the mucosa until dissolved. The application site may be buccal, sublingual, gingival or palatal, i.e. in a preferred embodiment, the administration of the transmucosal therapeutic system consists of applying the mucoadhesive layer structure to the buccal, sublingual, gingival or palatal mucosa, and preferably the buccal mucosa of the oral cavity of a human patient and maintaining the same on the mucosa until dissolved.

In certain embodiments, the transmucosal therapeutic system according to the invention provides a cumulative release of GLP-1 receptor agonist as measured with reconstructed human epithelial tissue of at least 0.002 mg/cm², at least 0.05 mg/cm² or at least 0.008 mg/cm², or less than or equal to 0.04 mg/cm², less than or equal to 0.03 mg/cm², or less than or equal to 0.025 mg/cm², or of from 0.002 mg/cm² to 0.04 mg/cm², from 0.005 mg/cm² to 0.03 mg/cm², or from 0.008 mg/cm² to 0.025 mg/cm² over a time period of 1 hour, and/or at least 0.005 mg/cm², at least 0.01 mg/cm² or at least 0.02 mg/cm², or less than or equal to 0.15 mg/cm², less than or equal to 0.1 mg/cm², or less than or equal to 0.07 mg/cm², or of from 0.005 mg/cm² to 0.15 mg/cm², from 0.01 mg/cm² to 0.1 mg/cm², or from 0.02 mg/cm² to 0.07 mg/cm² over a time period of 2 hours, and/or at least 0.01 mg/cm², at least 0.02 mg/cm² or at least 0.03 mg/cm², or less than or equal to 0.3 mg/cm², less than or equal to 0.2 mg/cm², or less than or equal to 0.1 mg/cm², or of from 0.01 mg/cm² to 0.3 mg/cm², from 0.02 mg/cm² to 0.2 mg/cm², or from 0.03 mg/cm² to 0.1 mg/cm² over a time period of 3 hours, and/or at least 0.02 mg/cm², at least 0.05 mg/cm² or at least 0.08 mg/cm², or less than or equal to 0.4 mg/cm², less than or equal to 0.3 mg/cm², or less than or equal to 0.25 mg/cm², or of from 0.02 mg/cm² to 0.4 mg/cm², from 0.05 mg/cm² to 0.3 mg/cm², or from 0.08 mg/cm² to 0.25 mg/cm² over a time period of 5 hours.

According to certain embodiments, the transmucosal therapeutic system according to the invention as described above provides a total amount permeated of the GLP-1 receptor agonist of at least 10 µg, at least 50 µg, or at least 100 µg per period of application. Also, according to certain embodiments, the transmucosal therapeutic system according to the invention as described above provides a total amount permeated of the GLP-1 receptor agonist of 500 µg or less, 300 µg or 150 µg or less per period of administration. In particular, according to certain embodiments, the transmucosal therapeutic system according to the invention as described above provides a total amount permeated of the GLP-1 receptor agonist of from 10 µg to 500 µg or less, from 50 µg to 350 µg or less, or from 100 µg to 200 µg or less, such as about 140 µg, per period of application. The period of application may be from about 10 minutes to about 5 hours, from about 30 minutes to about 3 hours, from about 45 minutes to about 2 hours, or from about 50 minutes to about 90 minutes

### METHOD OF TREATMENT/ MEDICAL USE

In accordance with a specific aspect of the present invention, the transmucosal therapeutic system according to the invention is for use in a method of treatment, and in particular in a method of treating a human patient. In accordance with another aspect, the present invention is related to a method of treatment, wherein the transmucosal therapeutic system according to the invention is administered to a human patient. In yet another aspect, the present invention relates to the use of the inventive transmucosal therapeutic system for the manufacture of a medicament for a treatment, preferably for the treatment of a human patient.

While GLP-1 receptor agonists are approved for the treatment of type 2 diabetes mellitus and, in some cases, obesity, treatment of other indications such as type 1 diabetes mellitus, hypoglycemia, cardiovascular disease, chronic kidney disease, liver diseases, gastrointestinal disorders, metabolic dysfunctions, skeletal muscle and bone conditions, and neurogenerative conditions has been suggested.

Thus, in certain embodiments, the transmucosal therapeutic system according to the invention is for use in a method of treating or preventing diabetes, in particular type 2 diabetes mellitus. Likewise, in certain other embodiments, the invention is related to a method of treating or preventing diabetes, in particular type 2 diabetes mellitus, wherein the transmucosal therapeutic system according to the invention is administered to a human patient. In yet other embodiments, the present invention relates to the use of the transmucosal therapeutic system according to the invention for the manufacture of a medicament for treating or preventing diabetes, in particular type 2 diabetes mellitus.

Further, in certain embodiments, the transmucosal therapeutic system according to the invention is for use in a method of treating or preventing obesity, in particular supporting weight management, including weight loss or weight maintenance. Likewise, in certain other embodiments, the invention is related to a method of treating or preventing obesity, in particular supporting weight management, including weight loss or weight maintenance, wherein the transmucosal therapeutic system according to the invention is administered to a human patient. In yet other embodiments, the present invention relates to the use of the transmucosal therapeutic system according to the invention for the manufacture of a medicament for treating or preventing obesity, in particular supporting weight management, including weight loss or weight maintenance.

The treatment of diabetes and/or obesity may include treatment of other (related) conditions such as those outlined above.

In addition, according to certain embodiments, the transmucosal therapeutic system according to the invention is for use in a method of treating a condition selected from the group consisting of hypoglycemia, cardiovascular disease, chronic kidney disease, liver diseases, gastrointestinal disorders, metabolic dysfunctions, skeletal muscle and bone conditions, and neurogenerative conditions. Likewise, in certain other embodiments, the invention is related to a method of treating a condition selected from the group consisting of hypoglycemia, cardiovascular disease, chronic kidney disease, liver diseases, gastrointestinal disorders, metabolic dysfunctions, skeletal muscle and bone conditions, and neurogenerative conditions, wherein the transmucosal therapeutic system according to the invention is administered to a human patient. In yet other embodiments, the present invention relates to the use of the transmucosal therapeutic system according to the invention for the manufacture of a medicament for treating a condition selected from the group consisting of hypoglycemia, cardiovascular disease, chronic kidney disease, liver diseases, gastrointestinal disorders, metabolic dysfunctions, skeletal muscle and bone conditions, and neurogenerative conditions.

Also, in a certain embodiment, treatment with the transmucosal therapeutic system according to the invention provides a reduction in at least one GLP-1 receptor agonist-related side effect relative to an equivalent oral (or subcutaneous) dose of the GLP-1 receptor agonist. As outlined above, in certain specific embodiments, such GLP-1 receptor agonist-related side effect is caused by passage through the gastrointestinal system and/or hepatic metabolism. Relative to an equivalent oral dose of a GLP-1 receptor agonist should be understood as a comparison in the incidence and intensity of side effects in a clinical study when using a dose of transmucosal and oral GLP-1 receptor agonist that leads substantially to the same blood plasma exposure of the GLP-1 receptor agonist. The incidence of the at least one GLP-1 receptor agonist-related side effect relative to an equivalent oral dose of the GLP-1 receptor agonist may be reduced by at least about 30 %, preferably at least about 40 %, more preferably at least about 70 % and most preferably at least about 80 %, and/or the intensity of the at least one GLP-1 receptor agonist-related side effect relative to an equivalent oral dose of GLP-1 receptor agonist may be reduced. The intensity of a side effect can be determined e.g. by classifying the side effects on a scale indicating "mild", "moderate" or "severe" intensity, and a reduction of the intensity can be quantified by comparing the median intensity.

The transmucosal therapeutic system according to the present invention is preferably administered to a human patient for a period of application of from about 10 minutes to about 5 hours, from about 30 minutes to about 3 hours, from about 45 minutes to about 2 hours, or from about 50 minutes to about 90 minutes.

In any of the treatments outlined for the above aspects and embodiments, the transmucosal therapeutic system is preferably administered by applying the mucoadhesive layer structure to the mucosa of the oral cavity of a human patient and maintained on the mucosa until dissolved. In preferred embodiments, the transmucosal therapeutic system is administered by applying the mucoadhesive layer structure to the buccal, sublingual, gingival or palatal mucosa of the oral cavity of a human patient and maintained on the mucosa until dissolved.

### PROCESS OF MANUFACTURE

The invention further relates to a process of manufacture of an GLP-1 receptor agonist-containing layer for use in a transmucosal therapeutic system and a corresponding mucoadhesive layer structure comprising the active agent-containing layer and a corresponding transmucosal therapeutic system.

In accordance with the invention, the process of manufacture of an active agent-containing layer of a transmucosal therapeutic system according to the invention comprises the steps of:
(a) combining at least the GLP-1 receptor agonist, the combination of permeation enhancers and the dissolvable film-forming agent in a solvent to obtain a coating composition;
(b) coating the coating composition onto a release liner; and
(c) drying the coated coating composition to form the active agent-containing layer.

In such a process, suitable GLP-1 receptor agonists, permeation enhancers and dissolvable film-forming agents are the same as those mentioned previously.

Thus, the GLP-1 receptor agonist used in the process of manufacture of an active agent-containing layer of a transmucosal therapeutic system according to the invention is not particularly limited as long as it is suitable for transmucosal delivery. In certain embodiments, the GLP-1 receptor agonist is selected from the group consisting of albiglutide, beinaglutide, cotadutide, dulaglutide, efinopegdutide, efocipegtrutide, efpeglenatide, exenatide, liraglutide, lixisenatide, pemvidutide, retatrutide, semaglutide, survodutide, taspoglutide, and tirzepatide.

The combination of permeation enhancers used in the process of manufacture of an active agent-containing layer of a transmucosal therapeutic system according to the invention is a combination of
i) a first permeation enhancer selected from the group consisting of C6 to C12 fatty acids and salts thereof; and
ii) a second permeation enhancer selected from the group consisting of bile acids and salts thereof.

According to certain embodiments,
i) the first permeation enhancer is selected from the group consisting of caproic acid, heptanoic acid, caprylic acid, nonanoic acid, capric acid, undecanoic acid, lauric acid and salts thereof. In certain specific embodiments, the first permeation enhancer is selected from the group consisting of caproic acid, caprylic acid, capric acid, lauric acid and salts thereof; and
ii) the second permeation enhancer is selected from the group consisting of cholic acid, taurocholic acid, glycocholic acid, deoxycholic acid, taurodeoxycholic acid, glycodeoxycholic acid, chenodeoxycholic acid, taurochenodeoxycholic acid, glycochenodeoxycholic acid, and salts thereof.

The dissolvable film-forming agent used in the process of manufacture of an active agent-containing layer of a transmucosal therapeutic system according to the invention is not particularly limited as long as it is "dissolvable".

The film-forming agent is not particularly limited as long as it is "dissolvable". In certain specific embodiments, the dissolvable film-forming agent is selected from the group consisting of polymers such as polyvinylpyrrolidone, methyl cellulose, ethyl cellulose, hydroxyethyl cellulose, hydroxypropyl cellulose, hydroxypropyl methyl cellulose, carboxymethyl cellulose sodium, polyethylene glycol- polyvinyl acetate- and polyvinylcaprolactame-based graft copolymers, polyvinyl alcohol, polyvinyl alcohol-polyethylene glycol copolymers, polyvinylpyrrolidone-polyvinylacetate copolymers, polyethylene oxides, polyethylene glycols, methacrylic acid - methyl methacrylate copolymers, and methacrylic acid - ethyl methacrylate copolymers, natural film-forming agents such as shellac, pectin, gelatine, alginate, pullulan and starch derivatives, and any mixtures thereof.

The preferences for the permeation enhancers and the dissolvable film-forming agent and other constituents of the active agent-containing layer are as outlined above. Thus, in certain specific embodiments, step a) consists of combining at least the GLP-1 receptor agonist, the combination of permeation enhancers, the dissolvable film-forming agent, and one or more excipients selected from the group consisting of taste masking agents, sweeteners, and flavoring agents, in a solvent to obtain a coating composition.

In step b), the coating composition may be coated onto the release liner keeping the corresponding film applicator gap at 300 µm or more and 3000 µm or less, at 500 µm or more and 2500 µm or less, or at 1000 µm or more and 1500 µm or less, such as at approx.. 1300 µm.

In step c), drying may be performed for at least 30 minutes, or at least 45 minutes, in one or more cycles at room temperature and/or at a temperature of from 30 to 70 °C, or from 40 to 60 °C, such as 50 °C.

The solvent used in each of the steps is not particularly limited, but in view of ease of coating, it is preferred that the solvent is able to dissolve substantially all of the layer components. In certain embodiments, the solvent comprises water.

A mucoadhesive layer structure comprising the active agent-containing layer and a corresponding transmucosal therapeutic system can be manufactured using the above-outlined process, using further manufacturing steps such as punching out individual transmucosal therapeutic system and packaging, e.g. by sealing in a pouch of a primary packaging material, as known to the skilled person. Such further steps preferably lead to a mucoadhesive layer structure or a transmucosal therapeutic system as described in the previous chapters.

The present invention in particular also relates to active agent-containing layers as well as mucoadhesive layer structures and transmucosal therapeutic system obtainable (and/or obtained) by the above-described processes.

### EXAMPLES

The present invention will now be more fully described with reference to the accompanying examples. It should be understood, however, that the following description is illustrative only and should not be taken in any way as a restriction of the invention. Numerical values provided in the examples regarding the amount of ingredients in the composition or the area weight may vary slightly due to manufacturing variability.

### PRE-EXAMPLES 1A-G

First permeation studies were performed with solutions comprising semaglutide without enhancer, with an enhancer selected from salcaprozate sodium, sodium caprate, or sodium glycocholate, or an enhancer combination of sodium caprate and sodium glycocholate in different weight ratios of enhancer to GLP-1 receptor agonist.

### Semaglutide-containing solution

Artificial saliva is prepared by dissolving 2.0 g potassium cyanate, 14.0 g potassium chloride, 1.8 g sodium dihydrogen phosphate monohydrate, and 0.548 g disodium hydrogen phosphate dihydrate in 1000 ml aqua purificata, adjusting the pH to 7.0 ± 0.05, and diluting the solution 1: 10 with aqua purificata. A stock solution of semaglutide in artificial saliva is prepared by dissolving 50.07 mg semaglutide in 50 ml artificial saliva to obtain a semaglutide stock solution with a concentration of 1.0 mg/mL.

The semaglutide-containing solutions to be used for the *in vitro* measurement are prepared by dissolving the enhancer(s) in the solution of semaglutide in artificial saliva as indicated in the tables summarizing the corresponding formulation below.

The formulations of the semaglutide-containing solutions of Pre-Examples 1a to 1e based on artificial saliva are summarized in Tables 1.1 and 1.2 below.

**Table 1.1**

| **Ingredient (Trade Name)** | **Pre-Ex. 1a** | **Pre-Ex. 1b** | **Pre-Ex. 1c** | **Pre-Ex. 1d** |
|---|---|---|---|---|
| Semaglutide stock solution (1.0 mg/mL) | N/A (solution used as is) | 2 mL | 4 mL | 4 mL |
| Salcaprozate sodium | - | 120.14 mg | - | - |
| Sodium caprate | - | - | 40.67 mg | 119.59 mg |
| Sodium glycocholate | - | - | - | - |
| | | | | |
| Ratio (GLP-1 : enhancer 1 : enhancer 2) | - | 1:60:0 | 1:10:0 | 1:30:0 |
| | | | | |

| Parameters of *in vitro* measurement | | | | |
|---|---|---|---|---|
| Volume applied | 250 µl | | | |
| Semaglutide amount | 234.32 µg | | | |
| applied (regarding 93.6% assay content) | | | | |
| Diffusion area | 0.6 cm² | | | |
| Semaglutide content applied | 390.5 µg/cm² | | | |

**Table 1.2**

| **Ingredient (Trade Name)** | **Pre-Ex. 1e** | **Pre-Ex. 1f** | **Pre-Ex. 1g** |
|---|---|---|---|
| Semaglutide stock solution (1.0 mg/mL) | 20 mL | 4 mL | 4 mL |
| Sodium caprate | - | - | 119.59 mg |
| Sodium glycocholate | 19.83 mg | 20.59 mg- | 21.08 mg- |
| | | | |
| Ratio (GLP-1 : enhancer) | 1:0:1 | 1:0:5 | 1:30:5 |
| | | | |

| Parameters of *in vitro* measurement | | | |
|---|---|---|---|
| Volume applied | 250 µl | | |
| Semaglutide amount applied (regarding 93.6% assay content) | 234.32 µg | | |
| Diffusion area | 0.6 cm² | | |
| Semaglutide content applied | 390.5 µg/cm² | | |

### Measurement of permeated amount

The permeated amount of the solutions according to Pre-Examples 1a to 1g was determined by *in vitro* experiments in accordance with the OECD Guideline (adopted April 13, 2004) using reconstructed human epithelial tissue inserts (human oral keratinocytes, EpiOral^{™} cell cultures). 6-well Labsolute^{®} cell culture plates were used as acceptor vessels, filled with 1.0 mL of acceptor medium (phosphate buffer solution pH 7.4 with 0.1 % (w/v%) sodium azide as antibacteriological agent). The tissue inserts had a diffusion area of 0.6 cm², and the bottom side of the tissue insert made full contact with the acceptor medium. 250 µl semaglutide-containing solution of each of Pre-Examples 1a to 1g was applied to the top side of the reconstructed tissue. Sampling was performed as full exchange of acceptor medium against fresh and prewarmed medium at sampling points. The permeated amount of semaglutide in the acceptor medium at a temperature of 37 ± 1°C (body temperature) was measured.

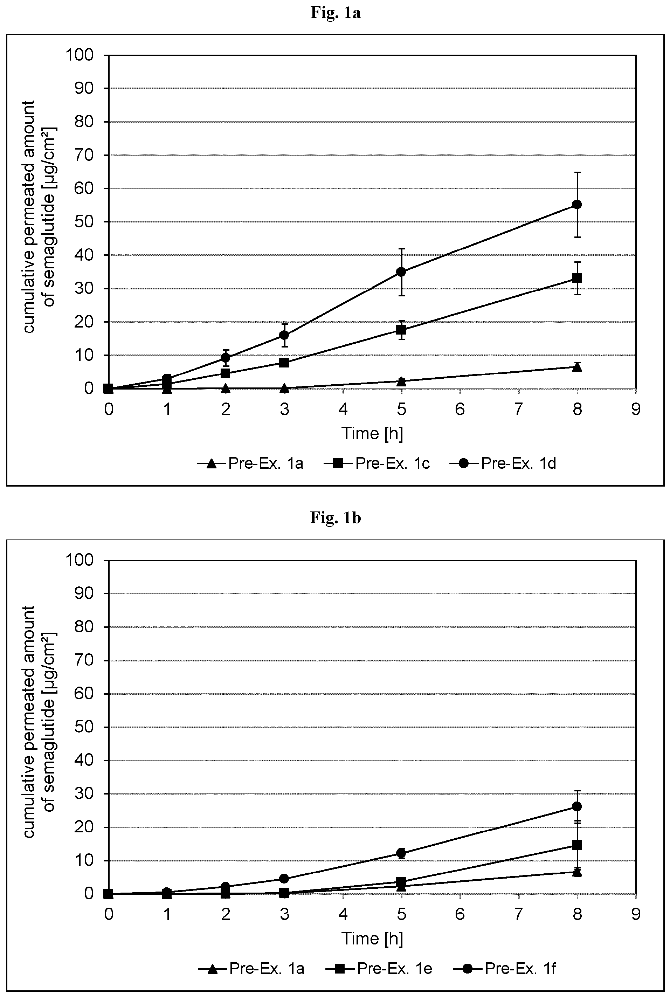

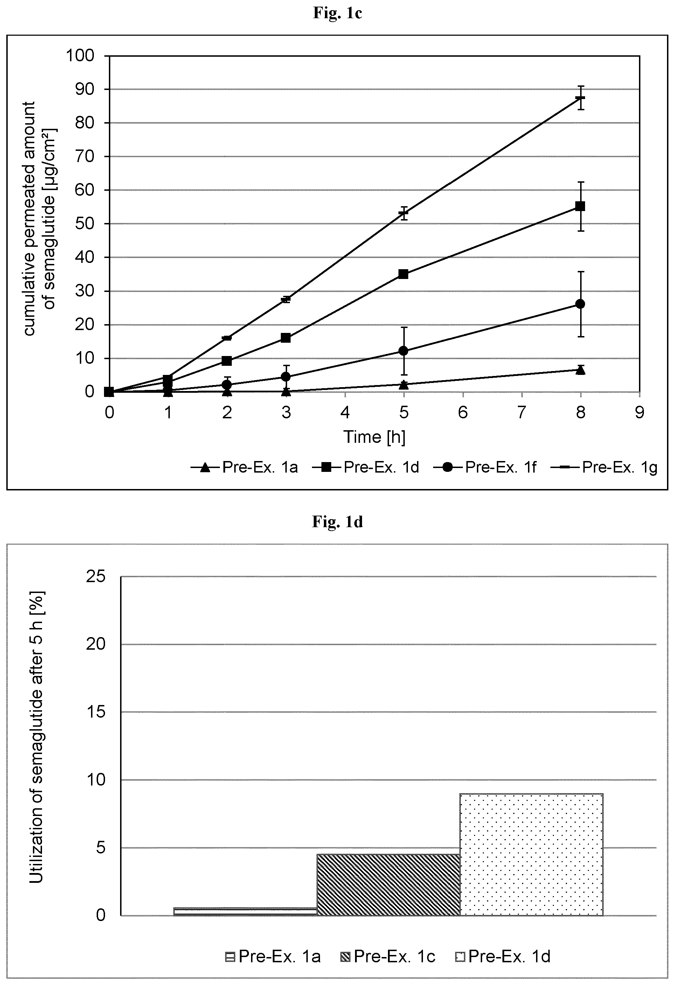

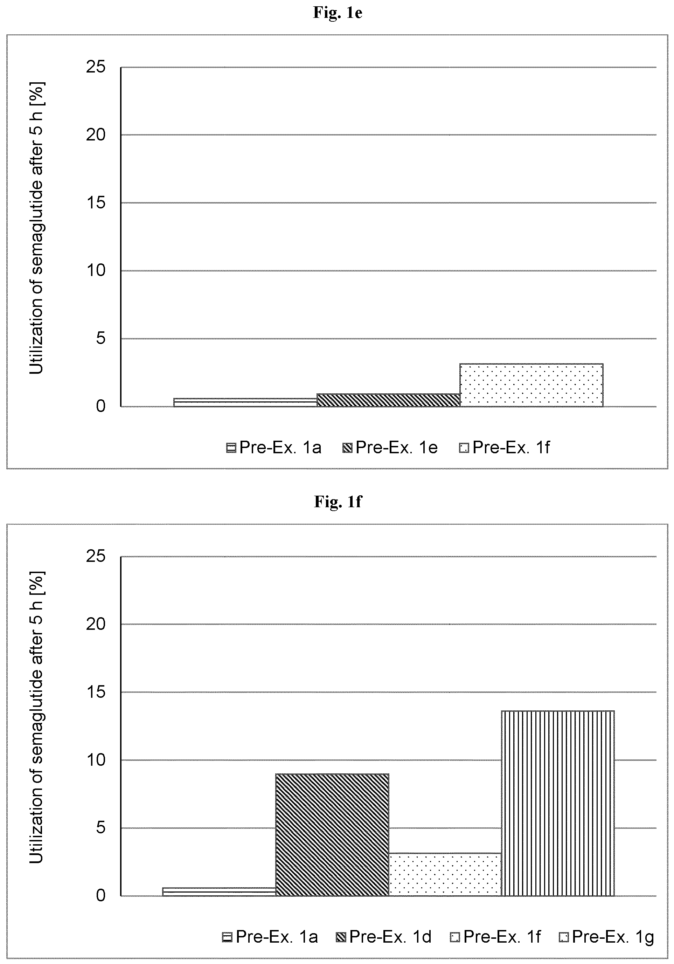

Pre-Example 1b did not show significant permeated amounts of semaglutide. The results of Pre-Examples 1a and 1c to 1g are shown in Tables 1.3 and 1.4 and Figures 1a to 1c. The results of Pre-Examples 1c compared to 1d as well as 1e compared to 1f show that increasing the amount of enhancer from 1:10:0 to 1:30:0 for sodium caprate or from 1:0:1 to 1:0:5 for sodium glycocholate increases the permeated amount of semaglutide, however, not proportionally (less than 100% increase for 3 to 5 times of the enhancer). The results obtained with Pre-Example 1g on the other hand surprisingly showed that the permeated amount could be proportionally increased by combining the increased amounts of enhancer of Pre-Examples 1d and 1f (see Fig. 1c).

**Table 1.3**

| **Cumulative permeated amount of semaglutide with SD* [µg/cm²]** | | | | | | |
|---|---|---|---|---|---|---|
| **Elapsed time [h]** | **Pre-Ex. 1a (n = 3)** | | **Pre-Ex. 1c (n = 3)** | | **Pre-Ex. 1d (n = 3)** | |
| | **Amt** | **SD** | **Amt** | **SD** | **Amt** | **SD** |
| **1** | 0.00 | 0.00 | 0.00 | 0.00 | 0.00 | 0.00 |
| **2** | 0.00 | 0.00 | 1.40 | 0.08 | 2.93 | 1.23 |
| **3** | 0.19 | 0.00 | 4.60 | 0.37 | 9.20 | 2.39 |
| **4** | 0.19 | 0.10 | 7.85 | 1.05 | 16.02 | 3.46 |
| **5** | 2.22 | 0.69 | 17.61 | 2.81 | 35.01 | 7.07 |

| | | | | | | |
|---|---|---|---|---|---|---|
| *: Standard deviation was calculated based on the n-method. | | | | | | |

**Table 1.4**

| **Cumulative permeated amount of semaglutide with SD* [µg/cm²]** | | | | | | |
|---|---|---|---|---|---|---|
| **Elapsed time [h]** | **Pre-Ex. 1e (n = 3)** | | **Pre-Ex. 1f (n = 3)** | | **Pre-Ex. 1g (n = 3)** | |
| | **Amt** | **SD** | **Amt** | **SD** | **Amt** | **SD** |
| **1** | 0.00 | 0.00 | 0.00 | 0.00 | 0.00 | 0.00 |
| **2** | 0.00 | 0.00 | 0.49 | 0.39 | 4.56 | 0.24 |
| **3** | 0.00 | 0.00 | 2.13 | 0.51 | 16.06 | 0.50 |
| **4** | 0.31 | 0.13 | 4.47 | 0.68 | 27.54 | 0.91 |
| **5** | 3.57 | 0.64 | 12.18 | 1.39 | 53.20 | 1.91 |

| | | | | | | |
|---|---|---|---|---|---|---|
| *: Standard deviation was calculated based on the n-method. | | | | | | |

### Utilization of semaglutide

The utilization of semaglutide at 5 hours was calculated based on the cumulative permeated amount at 5 hours and the initial semaglutide content. The results of Pre-Examples 1a and 1c to 1g are shown in Table 1.5 and Figures 1d to 1f.

**Table 1.5**

| **Utilization of semaglutide after 5 hours [%]** | | | | | |
|---|---|---|---|---|---|
| **Pre-Ex. 1a (n = 3)** | **Pre-Ex. 1c (n = 3)** | **Pre-Ex. 1d (n = 3)** | **Pre-Ex. 1e (n = 3)** | **Pre-Ex. 1f (n = 3)** | **Pre-Ex. 1g (n = 3)** |
| **0.57** | **4.51** | **8.97** | **0.91** | **3.12** | **13.62** |

The *in vitro* experiments show that using the combination of two enhancers (sodium caprate and sodium glycocholate) results in increased permeability, adding the single effect of each of the two enhancers, while increasing the amount of one enhancer does not result in linear increase of effect. In addition, the results of Pre-Example 1g are promising with regard to developing semaglutide transmucosal therapeutic systems of useful dimension, as an area of release of approx. 5 cm² would allow to provide the maximum daily dose of 140 µg semaglutide within 3 hours, assuming a similar semaglutide content of about 400 µg/cm².

### PRE-EXAMPLE 2A AND EXAMPLES 2B-D

Further permeation studies were performed with semaglutide-containing solution comprising an enhancer combination of sodium caprate and sodium glycocholate in a weight ratio of first permeation enhancer to second permeation enhancer to GLP-1 receptor agonist of 5:5:1 and with basic transmucosal therapeutic systems comprising 5.58 wt-% semaglutide and having a weight ratio of first permeation enhancer to second permeation enhancer to GLP-1 receptor agonist of 5:5:1. For the transmucosal therapeutic systems, different contents of semaglutide were tested by using one sample (single dose) or multiple samples stacked on top of each other, namely two samples (double dose) or three samples (triple dose).

### Semaglutide-containing solution (Pre-Example 2a)

Artificial saliva is prepared as described in Example 1 and the semaglutide-containing solution to be used for the *in vitro* measurement is prepared in accordance with the formulation of the solution as specified in Table 2.1 below.

The formulation of the semaglutide-containing solution of Pre-Example 2a based on artificial saliva is summarized in Table 2.1 below.

**Table 2.1**

| **Ingredient (Trade Name)** | **Pre-Ex. 2a** |
|---|---|
| Semaglutide | 30.30 mg |
| Sodium caprate | 150.63 mg |
| Sodium glycocholate | 149.74 mg |
| Artificial saliva | 29926.84 mg |
| | |
| Ratio (GLP-1 : enhancer 1 : enhancer 2) | 1:5:5 |
| | |

| Parameters of *in vitro* measurement | |
|---|---|
| Volume applied | 250 µl |
| Semaglutide amount applied (regarding 93.6% assay content) | 236.34 µg |
| Diffusion area | 0.6 cm² |
| Semaglutide content applied | 393.9 µg/cm² |

### Coating composition (Examples 2b, 2c, 2d)

The formulation of the semaglutide-containing coating composition of Examples 2b to 2d is summarized in Table 2.2 below. The formulation is based on weight percent, as also indicated in Table 2.2.

**Table 2.2**

| **Ingredient (Trade Name)** | **Examples 2b, 2c, 2d** | | | |
|---|---|---|---|---|
| | **Amt [g]** | | **Solids [%]** | |
| Semaglutide | 0.40 | | 5.58 | |
| Hydroxy propyl methyl cellulose. Visc.: 3 mPa s (Pharmacoat^{®} 603) | 0.67 | | 9.30 | |
| Hydroxy propyl methyl cellulose. Visc.: 15 mPa s (Pharmacoat^{®} 615) | 1.84 | | 25.57 | |
| Sodium caprate | 2.00 | | 27.78 | |
| Sodium glycocholate | 2.00 | | 27.78 | |
| PEG 400 | 0.29 | | 4.00 | |
| Total | 7.20 | | 100.01 | |
| Area weight [g/m²] | 79.7 | | | |
| Semaglutide content [µg/cm²] | 442.1 | | | |
| Ratio (GLP-1 : enhancer 1: enhancer 2) | 1:5:5 | | | |
| | | | | |

| | **Ex. 2b** | **Ex. 2c** | | **Ex. 2d** |
|---|---|---|---|---|
| Dose (semaglutide content) | single (442.1 µg/cm²) | double (884.1 µg/cm²) | | triple (1326.2 µg/cm²) |

### Preparation of the coating composition

For Example 2b, semaglutide and the two enhancers (sodium caprate and sodium glycocholate) were dissolved in aqua purificata while stirring. Hydroxy propyl methyl cellulose (Visc.: 3 mPa s and Visc.: 15 mPa s) and PEG 400 were added consecutively under stirring (420 rpm). The mixture was stirred for approx. 50 min.

### Coating of the coating composition

The resulting semaglutide-containing coating composition according to Example 2b was coated on the non-siliconized side of coating foil paper of 120 g/m² PE2 AB1 (which may function as release liner) and dried in the oven for 16 min at 50°, and in the climate chamber for 60 min at 25° and 60% humidity. Depending on the target area weight the corresponding film applicator gap was kept at approx. 550 µm.

The coating thickness gave an area weight of 79.7 g/m² (Ex. 2b), as determined after normal drying (expecting a residual moisture content of approx. 5%).

### Preparation of the transmucosal therapeutic system

Individual transmucosal therapeutic systems were punched out from the resulting semaglutide-containing laminate and sealed into pouches of the primary packaging material as conventional in the art.

In specific embodiments, a transmucosal therapeutic system as described above can be provided with a further mucoadhesive layer of larger surface area, preferably with rounded corners, which is free of active agent. This is of advantage when the OTF, on the basis of its physical properties alone, does not adhere sufficiently to the mucosa and/or when the semaglutide-containing layer, for the purpose of avoiding waste, has pronounced corners (square or rectangular shapes).

### Measurement of permeated amount

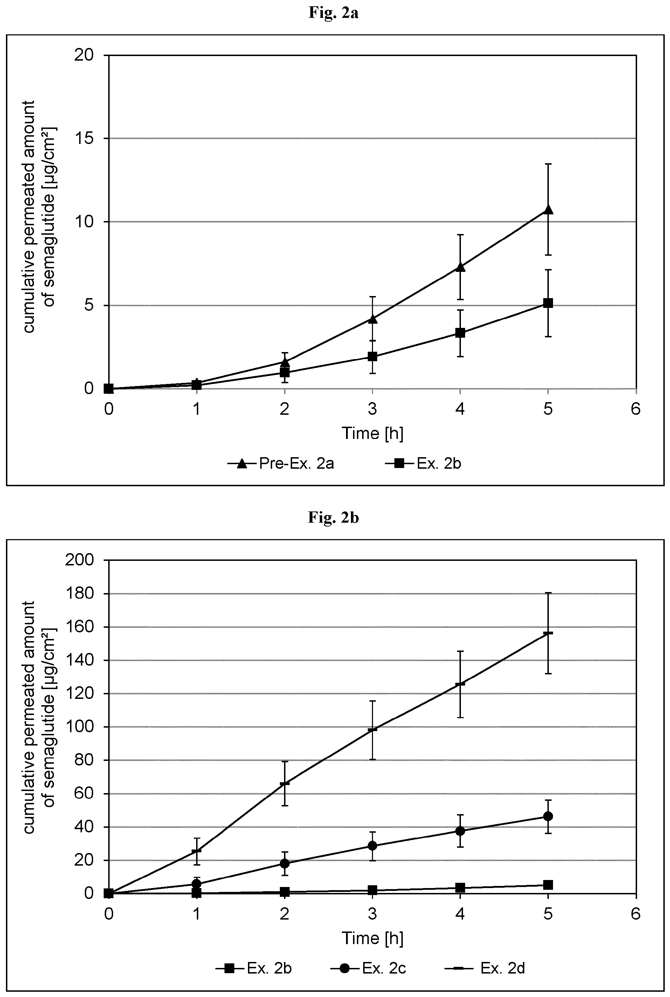

The permeated amount of the semaglutide-containing solution according to Pre-Example 2a and the transmucosal therapeutic systems prepared according to Examples 2b to 2d was determined by *in vitro* experiments in accordance with the OECD Guideline (adopted April 13, 2004) using reconstructed human epithelial tissue inserts (human oral keratinocytes, EpiOral^{™} cell cultures). 6-well Labsolute^{®} cell culture plates were used as acceptor vessels, filled with 1.0 mL of acceptor medium (phosphate buffer solution pH 7.4 with 0.1 % (w/v%) sodium azide as antibacteriological agent). The tissue inserts had a diffusion area of 0.6 cm², and the bottom side of the tissue insert made full contact with the acceptor medium. For Pre-Example 2a, 250 µl semaglutide-containing solution was applied to the top side of the reconstructed tissue. For Examples 2b to 2d, diecuts with an area of 0.527 cm² were punched from the transmucosal therapeutic system of Example 2b, stacked on top of each other for Example 2c (two diecuts) and Example 2d (three diecuts), as applicable, and applied to the top side of the reconstructed tissue with 125 µl artificial saliva each below and above the diecut(s). Sampling was performed as full exchange of acceptor medium against fresh and prewarmed medium at sampling points. The permeated amount of semaglutide in the acceptor medium at a temperature of 37 ± 1°C (body temperature) was measured. The results of Pre-Example 2a and Examples 2b to 2d are shown in Table 2.3 and Figures 2a and 2b.

**Table 2.3**

| **Cumulative permeated amount of semaglutide with SD* [µg/cm²]** | | | | | | | | |
|---|---|---|---|---|---|---|---|---|
| **Elapsed time [h]** | **Pre-Ex. 2a (n = 3)** | | **Ex. 2b (n = 3)** | | **Ex. 2c (n = 3)** | | **Ex. 2d (n = 3)** | |
| | **Amt** | **SD** | **Amt** | **SD** | **Amt** | **SD** | **Amt** | **SD** |
| **1** | 0.35 | 0.06 | 0.21 | 0.15 | 5.64 | 4.23 | 25.45 | 8.05 |
| **2** | 1.59 | 0.58 | 0.95 | 0.57 | 18.05 | 6.95 | 66.00 | 13.29 |
| **3** | 4.19 | 1.32 | 1.91 | 0.99 | 28.53 | 8.69 | 98.09 | 17.64 |
| **4** | 7.30 | 1.95 | 3.33 | 1.40 | 37.59 | 9.69 | 125.57 | 19.91 |
| **5** | 10.75 | 2.73 | 5.13 | 2.01 | 46.24 | 9.93 | 156.36 | 24.30 |

| | | | | | | | | |
|---|---|---|---|---|---|---|---|---|
| *: Standard deviation was calculated based on the n-method. Results are obtained with different lots of oral cell cultures, for which inter-lot variation is generally low enough so that results can be compared. Comparability of the data was also confirmed by internal control samples. | | | | | | | | |

### Utilization of semaglutide

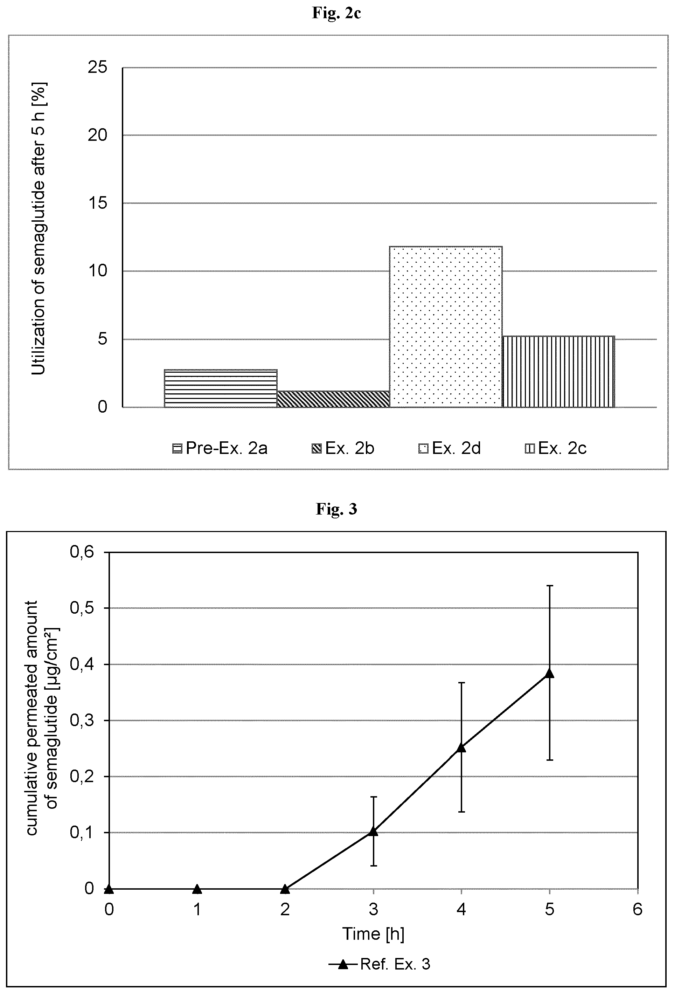

The utilization of semaglutide at 5 hours was calculated based on the cumulative permeated amount at 5 hours and the initial semaglutide content. The results of Pre-Example 2a and Examples 2b to 2d are shown in Table 2.4 and Figure 2c.

**Table 2.5**

| **Utilization of semaglutide after 5 hours [%]** | | | |
|---|---|---|---|
| **Ex. 2a (n = 3)** | **Ex. 2b (n = 3)** | **Ex. 2c (n = 3)** | **Ref. Ex. 2d (n = 3)** |
| **2.73** | **1.16** | **5.23** | **11.79** |

The *in vitro* experiments show similar permeability for the artificial saliva solution of Pre-Example 2a and the transmucosal therapeutic system of Example 2b having similar semaglutide contents (about 400 µg/cm²) and a weight ratio of first permeation enhancer to second permeation enhancer to GLP-1 receptor agonist of 5:5:1. Further, Examples 2b to 2d show that, with increasing area weight (and thus increasing active amount), the permeated amount also increases. In particular, the difference in the cumulative permeated amount of semaglutide between single dose, double dose and triple dose is higher than the difference in applied concentration and the correspondingly expected driving force due to the concentration gradient.

### REFERENCE EXAMPLE 3

Further comparative permeation studies were performed with transmucosal therapeutic systems comprising 3.75 wt-% semaglutide and no enhancers.

### Coating composition

The formulation of the semaglutide-containing coating composition of Reference Example 3 is summarized in Table 3.1 below. The formulations are based on weight percent, as also indicated in Table 3.1.

**Table 3.1**

| **Ingredient (Trade Name)** | **Ref. Ex. 3** | |
|---|---|---|
| | **Amt [g]** | **Solids [%]** |
| Semaglutide | 0.29 | 3.77 |
| Hydroxy propyl methyl cellulose. Visc.: 3 mPa s (Pharmacoat^{®} 603) | 2.75 | 36.06 |
| Hydroxy propyl methyl cellulose. Visc.: 80-120 mPa s | 3.16 | 41.40 |
| Hydroxy propyl methyl cellulose. Visc.: 100.000 mPa s (Benecel^{™} K100) | 0.13 | 1.66 |
| Sodium caprate | - | - |
| Sodium glycocholate | - | - |
| PEG400 | 0.29 | 3.83 |
| Glycerine | 0.49 | 6.35 |
| Mint oil | 0.23 | 3.03 |
| Eucalyptol | 0.13 | 1.71 |
| Bitter masker | 0.15 | 1.97 |
| Neotame | 0.02 | 0.22 |
| Total | 7.64 | 100.00 |
| Area weight [g/m²] | 219.5 | |
| Semaglutide content [µg/cm²] | 826.9 | |
| | | |
| Ratio (GLP-1 : enhancer 1: enhancer 2) | - | |

### Preparation of the coating composition

For Reference Example 3, Semaglutide was dissolved in aqua purificata while stirring at approx. 350 rpm. Hydroxy propyl methyl cellulose (Visc.: 3 mPa s, Visc.: 80-120 mPa s and Visc.: 100.000 mPa s), bitter masker and neotame were mixed and added under stirring at approx. 450 rpm. After at least 15 min, PEG 400, glycerine, mint oil and eucalyptol were added consecutively under stirring at approx. 200 rpm. The mixture was stirred for at least 2 hours and left to degas overnight.

### Coating of the coating composition

The resulting semaglutide-containing coating compositions according to Reference Example 3 was coated on the non-siliconized side of coating foil paper of 120 g/m² PE2 AB1 (which may function as release liner) and dried in the oven for 45 min at 50° (normal drying). Depending on the target area weight the corresponding film applicator gap was kept at approx. 1300 µm.

The coating thickness gave an area weight of 219.5 g/m² (Ref. Ex. 3a), as determined after total drying (expecting a residual moisture content of approx. 0%).

### Preparation of the transmucosal therapeutic system

See Example 2.

### Measurement of permeated amount

The permeated amount of the transmucosal therapeutic systems obtained according to

Reference Example 3 after normal drying was determined by *in vitro* experiments in accordance with the OECD Guideline (adopted April 13, 2004) using reconstructed human epithelial tissue inserts (human oral keratinocytes, EpiOral^{™} cell cultures). 6-well Labsolute^{®} cell culture plates were used as acceptor vessels, filled with 1.0 mL of acceptor medium (phosphate buffer solution pH 7.4 with 0.1 % (w/v%) sodium azide as antibacteriological agent). The tissue inserts had a diffusion area of 0.6 cm², and the bottom side of the tissue insert made full contact with the acceptor medium. Diecuts with an area of 0.527 cm² were punched from the transmucosal therapeutic systems of Reference Example 3 and applied to the top side of the reconstructed tissue with 125 µl artificial saliva each below and above the diecut. Sampling was performed as full exchange of acceptor medium against fresh and prewarmed medium at sampling points. The permeated amount of semaglutide in the acceptor medium at a temperature of 37 ± 1°C (body temperature) was measured. The results are shown in Table 3.2 and Figure 3.

**Table 3.2**

| **Cumulative permeated amount of semaglutide with SD* [µg/cm²]** | | |
|---|---|---|
| **Elapsed time [h]** | **Ref. Ex. 3 (n = 3)** | |
| | **Amt** | **SD** |
| **1** | 0.00 | 0.00 |
| **2** | 0.00 | 0.00 |
| **3** | 0.10 | 0.06 |
| **4** | 0.25 | 0.12 |
| **5** | 0.38 | 0.16 |

| | | |
|---|---|---|
| *: Standard deviation was calculated based on the n-method. | | |

### Utilization of semaglutide

The utilization of semaglutide at 5 hours was calculated based on the cumulative permeated amount at 5 hours and the initial semaglutide content. The result is shown in Table 3.3.

**Table 3.3**

| **Utilization of semaglutide after 5 hours [%]** |
|---|
| **Ref. Ex. 3 (n = 3)** |
| **0.00** |

### EXAMPLES 4A-B

Further permeation studies were performed with transmucosal therapeutic systems comprising 3 wt-% semaglutide and having a weight ratio of first permeation enhancer to second permeation enhancer to GLP-1 receptor agonist of 5:5: 1, or comprising 3.75 wt-% semaglutide and having a weight ratio of first permeation enhancer to second permeation enhancer to GLP-1 receptor agonist of 4:4:1.

### Coating composition

The formulations of the semaglutide-containing coating compositions of Examples 4a and 4b are summarized in Table 4.1 below. The formulations are based on weight percent, as also indicated in Table 4.1.

**Table 4.1**

| **Ingredient (Trade Name)** | **Ex. 4a** | | **Ex. 4b** | |
|---|---|---|---|---|
| | **Amt [g]** | **Solids [%]** | **Amt [g]** | **Solids [%]** |
| Semaglutide | 0.45 | 3.00 | 0.28 | 3.77 |
| Hydroxy propyl methyl cellulose. Visc.: 3 mPa s (Pharmacoat^{®} 603) | 3.91 | 25.98 | 1.71 | 22.83 |
| Hydroxy propyl methyl cellulose. Visc.: 80-120 mPa s | 3.49 | 23.19 | 1.93 | 25.78 |
| Hydroxy propyl methyl cellulose. Visc.: 100.000 mPa s (Benecel^{™} K100) | 0.15 | 1.02 | 0.08 | 1.07 |
| Sodium caprate | 2.25 | 14.97 | 1.13 | 15.02 |
| Sodium glycocholate | 2.26 | 14.99 | 1.13 | 15.06 |
| PEG400 | 0.58 | 3.86 | 0.30 | 4.06 |
| Glycerine | 0.94 | 6.23 | 0.44 | 5.84 |
| Mint oil | 0.43 | 2.86 | 0.22 | 2.90 |
| Eucalyptol | 0.25 | 1.68 | 0.11 | 1.47 |
| Bitter masker | 0.30 | 2.02 | 0.15 | 2.01 |
| Neotame | 0.03 | 0.20 | 0.02 | 0.20 |
| Total | 15.04 | 100.00 | 7.50 | 100.01 |
| Area weight [g/m²] | 209.7 | | 189.9 | |
| Semaglutide content [µg/cm²] | 628.7 | | 716.3 | |
| | | | | |
| Ratio (GLP-1 : enhancer 1: enhancer 2) | 1:5:5 | | 1:4:4 | |

### Preparation of the coating composition

For Examples 4a and 4b, the two enhancers (sodium caprate and sodium glycocholate) were dissolved in aqua purificata while stirring at approx.200 rpm. Semaglutide was added under stirring at approx. 350 rpm. Hydroxy propyl methyl cellulose (Visc.: 3 mPa s, Visc.: 80-120 mPa s and Visc.: 100.000 mPa s), bitter masker and neotame were mixed and added under stirring at approx. 450 rpm. After at least 15 min, PEG 400, glycerine, mint oil and eucalyptol were added consecutively under stirring at approx. 450 rpm. The mixture was stirred for at least 2 hours and left to degas overnight.

### Coating of the coating composition

The resulting semaglutide-containing coating compositions according to Examples 4a and 4b were coated on the non-siliconized side of coating foil paper of 120 g/m² PE2 AB1 (which may function as release liner) and dried in the oven for 45 min at 50° (normal drying). Depending on the target area weight the corresponding film applicator gap was kept at approx. 1300 µm.

The coating thickness gave an area weight of 209.7 g/m² (Ex. 4a), and 189.9 g/m² (Ex. 4b), respectively, as determined after total drying (expecting a residual moisture content of approx. 0%)..

### Preparation of the transmucosal therapeutic system

See Example 2.

### Measurement of permeated amount

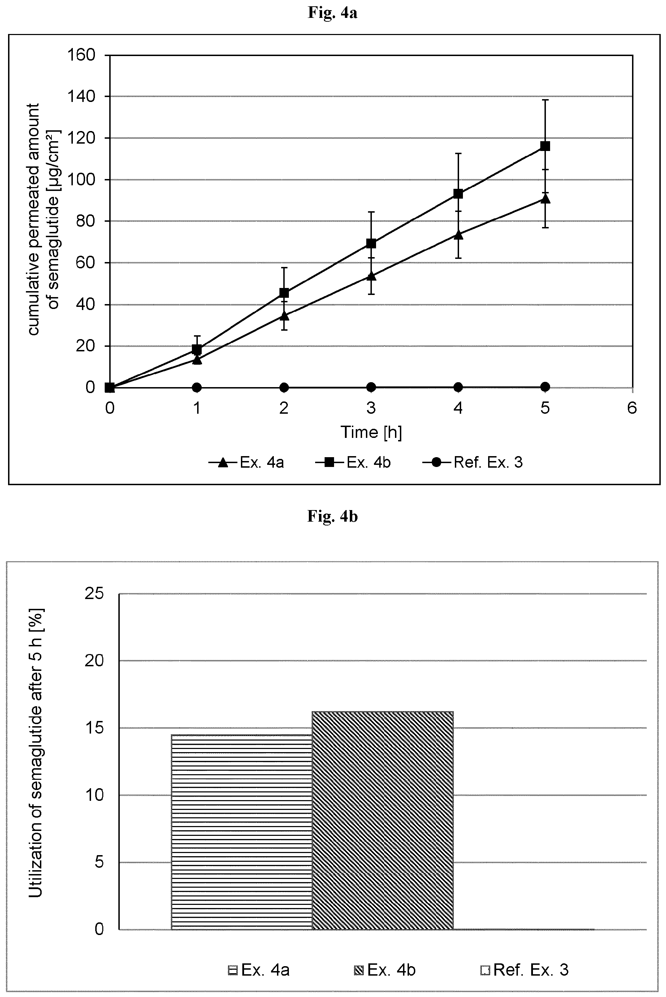

The permeated amount of the transmucosal therapeutic systems obtained according to Examples 4a and 4b after normal drying was determined by *in vitro* experiments in accordance with the OECD Guideline (adopted April 13, 2004) using reconstructed human epithelial tissue inserts (human oral keratinocytes, EpiOral^{™} cell cultures). 6-well Labsolute^{®} cell culture plates were used as acceptor vessels, filled with 1.0 mL of acceptor medium (phosphate buffer solution pH 7.4 with 0.1 % (w/v%) sodium azide as antibacteriological agent). The tissue inserts had a diffusion area of 0.6 cm², and the bottom side of the tissue insert made full contact with the acceptor medium. Diecuts with an area of 0.527 cm² were punched from the transmucosal therapeutic systems of Examples 4a and 4b and applied to the top side of the reconstructed tissue with 125 µl artificial saliva each below and above the diecut. Sampling was performed at full exchange of acceptor medium against fresh and prewarmed medium at sampling points. The permeated amount of semaglutide in the acceptor medium at a temperature of 37 ± 1°C (body temperature) was measured. The results are shown in Table 4.2 and Figure 4a.

**Table 4.2**

| **Cumulative permeated amount of semaglutide with SD* [µg/cm²]** | | | | |
|---|---|---|---|---|
| **Elapsed time [h]** | **Ex. 4a (n = 3)** | | **Ex. 4b (n = 3)** | |
| | **Amt** | **SD** | **Amt** | **SD** |
| **1** | 13.63 | 2.18 | 18.32 | 6.66 |
| **2** | 34.66 | 6.81 | 45.50 | 12.25 |
| **3** | 53.78 | 8.76 | 69.41 | 15.25 |
| **4** | 73.67 | 11.36 | 93.08 | 19.81 |
| **5** | 91.03 | 14.00 | 116.14 | 22.31 |

| | | | | |
|---|---|---|---|---|
| *: Standard deviation was calculated based on the n-method. | | | | |

### Utilization of semaglutide

The utilization of semaglutide at 5 hours was calculated based on the cumulative permeated amount at 5 hours and the initial semaglutide content. The results are shown in Table 4.3 and in Figure 4b.

**Table 4.3**

| **Utilization of semaglutide after 5 hours [%]** | |
|---|---|
| **Ex. 4a (n = 3)** | **Ex. 4b (n = 3)** |
| **14.48** | **16.21** |

### EXAMPLES 5A-5C

Further permeation studies were performed with transmucosal therapeutic systems comprising 3 wt-% or 5 wt-% semaglutide and having a weight ratio of first permeation enhancer to second permeation enhancer to GLP-1 receptor agonist of 4:4:1 or 3:3:1, respectively.

### Coating composition

The formulations of the semaglutide-containing coating compositions of Examples 5a to 5c are summarized in Table 5.1 below. The formulations are based on weight percent, as also indicated in Table 5.1.

**Table 5.1**

| **Ingredient (Trade Name)** | **Ex. 5a** | | **Ex. 5b** | | **Ex. 5c** | |
|---|---|---|---|---|---|---|
| | **Amt [g]** | **Solids [%]** | **Amt [g]** | **Solids [%]** | **Amt [g]** | **Solids [%]** |
| Semaglutide | 0.45 | 3.01 | 0.45 | 3.02 | 0.75 | 4.99 |
| Hydroxy propyl methyl cellulose. Visc.: 3 mPa s (Pharmacoat^{®} 603) | 4.37 | 29.03 | 4.83 | 32.13 | 3.75 | 24.86 |
| Hydroxy propyl methyl cellulose. Visc.: 80-120 mPa s | 3.91 | 25.98 | 4.33 | 28.80 | 3.36 | 22.30 |
| Hydroxy propyl methyl cellulose. Visc.: 100.000 mPa s (Benecel^{™} K100) | 0.18 | 1.18 | 0.19 | 1.29 | 0.15 | 1.00 |
| Sodium caprate | 1.80 | 11.94 | 1.36 | 9.01 | 2.25 | 14.94 |
| Sodium glycocholate | 1.80 | 11.97 | 1.35 | 8.99 | 2.25 | 14.96 |
| PEG400 | 0.61 | 4.04 | 0.59 | 3.94 | 0.60 | 3.97 |
| Glycerine | 0.92 | 6.10 | 0.91 | 6.05 | 0.92 | 6.09 |
| Mint oil | 0.45 | 3.00 | 0.45 | 2.96 | 0.48 | 3.19 |
| Eucalyptol | 0.23 | 1.51 | 0.24 | 1.61 | 0.23 | 1.51 |
| Bitter masker | 0.30 | 2.02 | 0.30 | 2.00 | 0.30 | 1.99 |
| Neotame | 0.03 | 0.22 | 0.03 | 0.20 | 0.03 | 0.21 |
| Total | 15.05 | 100.00 | 15.03 | 100.00 | 15.07 | 100.01 |
| Area weight [g/m²] | 206.5 | | 212.4 | | 203.8 | |
| Semaglutide content [µg/cm²] | 621.4 | | 641.2 | | 1015.9 | |
| | | | | | | |
| Ratio (GLP-1 : enhancer 1: enhancer 2) | 1:4:4 | | 1:3:3 | | | |

### Preparation of the coating composition

For Examples 5a to 5c, the two enhancers (sodium caprate and sodium glycocholate) were dissolved in aqua purificata while stirring at approx.200 rpm. Semaglutide was added under stirring at approx. 350 rpm. Hydroxy propyl methyl cellulose (Visc.: 3 mPa s, Visc.: 80-120 mPa s and Visc.: 100.000 mPa s), bitter masker and neotame were mixed and added under stirring at approx. 450 rpm. After at least 15 min, PEG 400, glycerine, mint oil and eucalyptol were added consecutively under stirring at approx. 450 rpm. The mixture was stirred for at least 2 hours and left to degas overnight.

### Coating of the coating composition

The resulting semaglutide-containing coating compositions according to Examples 5a to 5c were coated on the non-siliconized side of coating foil paper of 120 g/m² PE2 AB1 (which may function as release liner) and dried in the oven for 45 min at 50° (normal drying). Depending on the target area weight the corresponding film applicator gap was kept at approx. 1300 µm.

The coating thickness gave an area weight of 206.5 g/m² (Ex. 5a), 212.4 g/m² (Ex. 5b), and 203.8 g/m² (Ex. 5c), respectively, as determined after total drying (expecting a residual moisture content of approx. 0%.

### Preparation of the transmucosal therapeutic system

See Example 2.

### Measurement of permeated amount

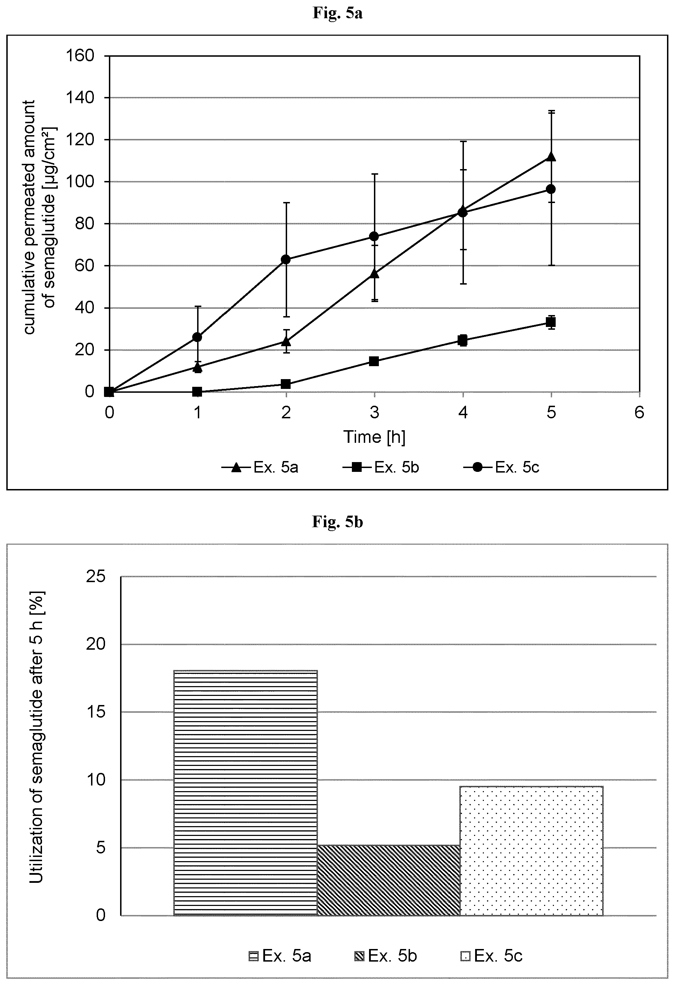

The permeated amount of the transmucosal therapeutic systems obtained according to Examples 5a to 5c after normal drying was determined by *in vitro* experiments in accordance with the OECD Guideline (adopted April 13, 2004) using reconstructed human epithelial tissue inserts (human oral keratinocytes, EpiOral^{™} cell cultures). 6-well Labsolute^{®} cell culture plates were used as acceptor vessels, filled with 1.0 mL of acceptor medium (phosphate buffer solution pH 7.4 with 0.1 % (w/v%) sodium azide as antibacteriological agent). The tissue inserts had a diffusion area of 0.6 cm², and the bottom side of the tissue insert made full contact with the acceptor medium. Diecuts with an area of 0.527 cm² were punched from the transmucosal therapeutic systems of Examples 5a to 5c and applied to the top side of the reconstructed tissue with 125 µl artificial saliva each below and above the diecut. Sampling was performed as full exchange of acceptor medium against fresh and prewarmed medium at sampling points. The permeated amount of semaglutide in the acceptor medium at a temperature of 37 ± 1°C (body temperature) was measured. The results are shown in Table 5.2 and Figure 5a.

**Table 5.2**

| **Cumulative permeated amount of semaglutide with SD* [µg/cm²]** | | | | | | |
|---|---|---|---|---|---|---|
| **Elapsed time [h]** | **Ex. 5a (n = 3)** | | **Ex. 5b (n = 3)** | | **Ex. 5c (n = 3)** | |
| | **Amt** | **SD** | **Amt** | **SD** | **Amt** | **SD** |
| **1** | 11.97 | 2.59 | 0.00 | 0.00 | 26.06 | 14.76 |
| **2** | 24.11 | 5.50 | 3.62 | 1.38 | 63.05 | 27.18 |
| **3** | 56.45 | 13.31 | 14.55 | 1.30 | 73.94 | 29.88 |
| **4** | 86.81 | 19.00 | 24.58 | 2.58 | 85.36 | 33.95 |
| **5** | 112.15 | 21.90 | 33.15 | 3.22 | 96.55 | 36.23 |

| | | | | | | |
|---|---|---|---|---|---|---|
| *: Standard deviation was calculated based on the n-method. | | | | | | |

### Utilization of semaglutide

The utilization of semaglutide at 5 hours was calculated based on the cumulative permeated amount at 5 hours and the initial semaglutide content. The results are shown in Table 5.3 and in Figure 5b.

**Table 5.3**

| Utilization of semaglutide after 5 hours [%] | | |
|---|---|---|
| Ex. 5a (n = 3) | Ex. 5b (n = 3) | Ex. 5c (n = 3) |
| 18.05 | 5.17 | 9.50 |

### EXAMPLE 6A, 6B AND REFERENCE EXAMPLE 6C

Further permeation studies were performed with transmucosal therapeutic systems comprising 3 wt-% tirzepatide or liraglutide, respectively, and having a weight ratio of first permeation enhancer to second permeation enhancer to GLP-1 receptor agonist of 5:5:1 (as applicable).

### Coating composition

The formulations of the tirzepatide- or liraglutide-containing coating compositions of Example 6a, 6b and Reference Example 6c are summarized in Table 6.1 below. The formulations are based on weight percent, as also indicated in Table 6.1.

**Table 6.1**

| **Ingredient (Trade Name)** | **Ex. 6a** | | **Ex. 6b** | | **Ref. Ex. 6c** | |
|---|---|---|---|---|---|---|
| | **Amt [g]** | **Solids [%]** | **Amt [g]** | **Solids [%]** | **Amt [g]** | **Solids [%]** |
| Tirzepatide | 0.22 | 3.00 | - | - | - | - |
| Liraglutide | - | - | 0.45 | 2.98 | 0.23 | 3.00 |
| Hydroxy propyl methyl cellulose. | 1.96 | 26.13 | 3.91 | 26.00 | 3.20 | 42.48 |
| Visc.: 3 mPa s (Pharmacoat^{®} 603) | | | | | | |
| Hydroxy propyl methyl cellulose. | 1.74 | 23.28 | 3.51 | 23.33 | 2.86 | 38.07 |
| Visc.: 80-120 mPa s | | | | | | |
| Hydroxy propyl methyl cellulose. | 0.08 | 1.01 | 0.15 | 0.98 | 0.12 | 1.57 |
| Visc.: 100.000 mPa s (Benecel^{™} K100) | | | | | | |
| Sodium caprate | 1.13 | 15.11 | 2.25 | 14.96 | - | - |
| Sodium glycocholate | 1.13 | 15.04 | 2.25 | 14.97 | - | - |
| PEG400 | 0.28 | 3.76 | 0.59 | 3.95 | 0.31 | 4.10 |
| Glycerine | 0.46 | 6.07 | 0.92 | 6.14 | 0.46 | 6.13 |
| Mint oil | 0.22 | 2.89 | 0.44 | 2.92 | 0.22 | 2.92 |
| Eucalyptol | 0.11 | 1.47 | 0.24 | 1.57 | 0.12 | 1.54 |
| Bitter masker | 0.15 | 2.01 | 0.30 | 1.99 | - | - |
| Neotame | 0.02 | 0.21 | 0.03 | 0.21 | 0.01 | 0.20 |
| Total | 7.50 | 99.98 | 14.59 | 100.00 | 7.53 | 100.01 |
| Area weight [g/m²] | 187.1 | | 206.4 | | 230.5 | |
| Tirzepatide content [µg/cm²] | 560.2 | | 613.8 | | 691.0 | |
| | | | | | | |
| Ratio (GLP-1: enhancer 1: enhancer 2) | 1:5:5 | | | | - | |

### Preparation of the coating composition

For Examples 6a and 6b, the two enhancers (sodium caprate and sodium glycocholate) were dissolved in aqua purificata while stirring at approx.200 rpm. Tirzepatide or Liraglutide, respectively, was added under stirring at approx. 350 rpm. Hydroxy propyl methyl cellulose (Visc.: 3 mPa s, Visc.: 80-120 mPa s and Visc.: 100.000 mPa s), bitter masker and neotame were mixed and added under stirring at approx. 450 rpm. After at least 15 min, PEG 400, glycerine, mint oil and eucalyptol were added consecutively under stirring at approx. 450 rpm. The mixture was stirred for at least 2 hours and left to degas overnight.

For Reference Example 6c, Liraglutide was dissolved in aqua purificata while stirring at approx. 200 rpm. Hydroxy propyl methyl cellulose (Visc.: 3 mPa s, Visc.: 80-120 mPa s and Visc.: 100.000 mPa s) and neotame were mixed and added under stirring at approx. 150 rpm. After at least 15 min, PEG 400, glycerine, mint oil and eucalyptol were added consecutively under stirring at approx. 100 rpm. The mixture was stirred for at least 2 hours and left to degas overnight.

### Coating of the coating composition

The resulting tirzepatide-containing coating composition according to Example 6a and the resulting liraglutide-containing coating compositions according to Example 6b and Reference Example 6c were coated on the non-siliconized side of coating foil paper of 120 g/m² PE2 AB1 (which may function as release liner) and dried in the oven for 45 min at 50° (normal drying). Depending on the target area weight the corresponding film applicator gap was kept at approx. 1300 µm.

The coating thickness gave an area weight of 187.1 g/m² (Ex. 6a), 206.4 g/m² (Ex. 6b), and 230.5 g/m² (Ref. Ex. 6c), respectively, as determined after total drying (expecting a residual moisture content of approx. 0%).

### Preparation of the transmucosal therapeutic system

See Example 2.

### Measurement of permeated amount

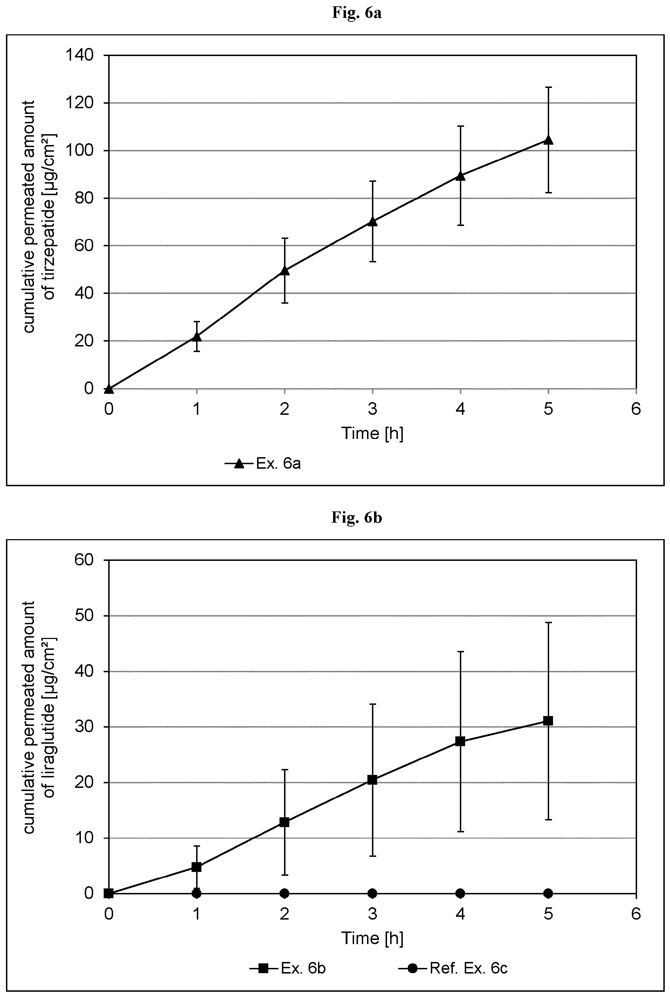

The permeated amount of the transmucosal therapeutic systems obtained according to Example 6a, 6b and Reference Example 6c after normal drying was determined by *in vitro* experiments in accordance with the OECD Guideline (adopted April 13, 2004) using reconstructed human epithelial tissue inserts (human oral keratinocytes, EpiOral^{™} cell cultures). 6-well Labsolute^{®} cell culture plates were used as acceptor vessels, filled with 1.0 mL of acceptor medium (phosphate buffer solution pH 7.4 with 0.1 % (w/v%) sodium azide as antibacteriological agent). The tissue inserts had a diffusion area of 0.6 cm², and the bottom side of the tissue insert made full contact with the acceptor medium. Diecuts with an area of 0.527 cm² were punched from the transmucosal therapeutic systems of Examples 6a, 6b and Reference Example 6c and applied to the top side of the reconstructed tissue with 125 µl artificial saliva each below and above the diecut. Sampling was performed as full exchange of acceptor medium against fresh and prewarmed medium at sampling points. The permeated amount of semaglutide in the acceptor medium at a temperature of 37 ± 1°C (body temperature) was measured. The results of Example 6a are shown in Table 6.2 and Figure 6a. The results of Example 6b and Reference Example 6c are shown in Table 6.3 and Figure 6b.

**Table 6.2**

| **Cumulative permeated amount of tirzepatide with SD* [µg/cm²]** | | |
|---|---|---|
| **Elapsed time [h]** | **Ex. 6a (n = 3)** | |
| | **Amt** | **SD** |
| **1** | 21.94 | 6.21 |
| **2** | 49.68 | 13.65 |
| **3** | 70.26 | 16.91 |
| **4** | 89.43 | 20.75 |
| **5** | 104.49 | 22.09 |

| | | |
|---|---|---|
| *: Standard deviation was calculated based on the n-method. | | |

**Table 6.3**

| **Cumulative permeated amount of liraglutide with SD* [µg/cm²]** | | | | |
|---|---|---|---|---|
| **Elapsed time [h]** | **Ex. 6b (n = 3)** | | **Ref. Ex. 6c (n = 3)** | |
| | **Amt** | **SD** | **Amt** | **SD** |
| **1** | 4.77 | 3.80 | 0.00 | 0.00 |
| **2** | 12.86 | 9.45 | 0.00 | 0.00 |
| **3** | 20.46 | 13.68 | 0.00 | 0.00 |
| **4** | 27.38 | 16.17 | 0.00 | 0.00 |
| **5** | 31.07 | 17.73 | 0.00 | 0.00 |

| | | | | |
|---|---|---|---|---|
| *: Standard deviation was calculated based on the n-method. Results are obtained with different lots of oral cell cultures, for which inter-lot variation is generally low enough so that results can be compared. Comparability of the data was also confirmed by internal control samples. | | | | |

### Utilization of tirzepatide and liraglutide

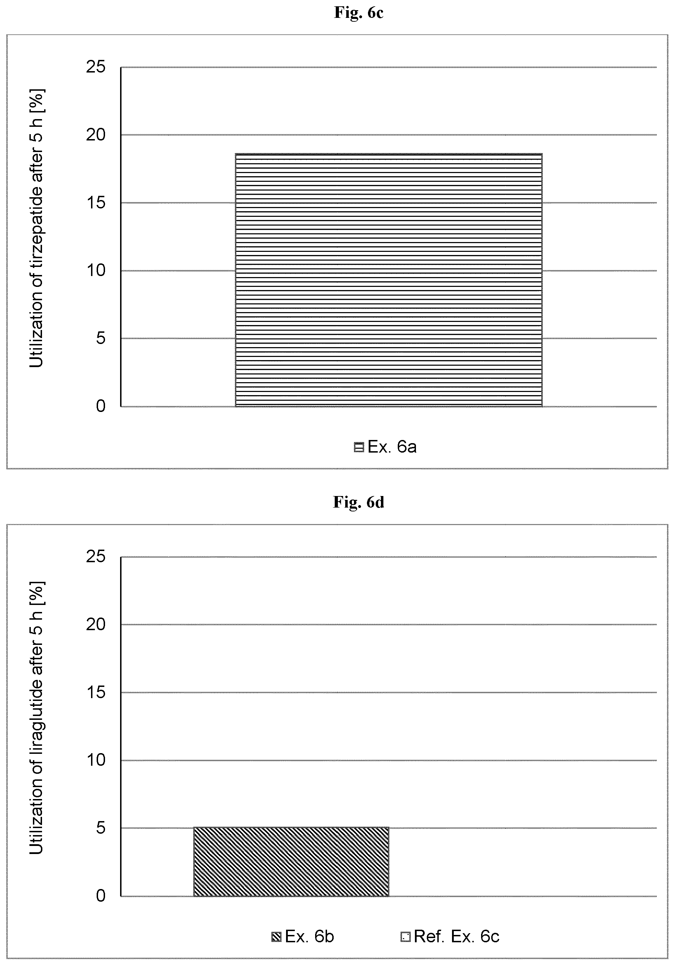

The utilization of tirzepatide or liraglutide, respectively, at 5 hours was calculated based on the cumulative permeated amount at 5 hours and the initial tirzepatide or liraglutide content. The results of Example 6a are shown in Table 6.4 and Figure 6c. The results of Example 6b and Reference Example 6c are shown in Table 6.5 and Figure 6d.

**Table 6.4**

| **Utilization of tirzepatide after 5 hours [%]** |
|---|
| **Ex. 6a (n = 3)** |
| **18.62** |

**Table 6.5**

| **Utilization of liraglutide after 5 hours [%]** | |
|---|---|
| **Ex. 6b (n = 3)** | **Ref. Ex. 6c (n = 3)** |
| **5.06** | **0.00** |

### SKIN IRRITATION POTENTIAL EXPERIMENT 7

For prediction of the irritation potential of the enhancers used in artificial saliva solution and transmucosal therapeutic systems, the reduction of the viability of tissues exposed to the enhancers in relation to a negative control was determined using a MTT cell viability assay.

The assay consists of an exposure of reconstructed human epithelial tissue inserts (human oral keratinocytes, EpiOral^{™} cell cultures) to the penetration enhancers followed by a cell viability test. Cell viability is measured by dehydrogenase conversion of MTT [(3-4,5-dimethyl thiazole 2-yl) 2,5-diphenyltetrazolium-bromide], present in cell mitochondria, into a blue formazan salt that is quantitatively measured after extraction from tissues.

Initial tests with recultivated reconstructed tissue used in the above permeation studies showed that increasing the amount of enhancer generally results in stronger reduction of the cell viability.

In order to predict the irritation potential of the specific enhancer combination (sodium caprate and sodium glycocholate) used in the transmucosal therapeutic systems described above, a MTT effective time (ET-50) test was performed with fresh EpiOral^{™} cell cultures being exposed to an enhancer solution providing the same concentration as a transmucosal therapeutic system comprising 15 wt-% of sodium caprate and of sodium glycocholate.

**The solutions used for the MTT ET-50 test are indicate below.**

| | |
|---|---|
| Test substance (TS): | 45 mg/ml sodium caprate and 45 mg/ml sodium glycocholate in artificial saliva (corresponding to 15 wt-% each, based on a transmucosal therapeutic system with an area weight of 200 g/m²) |
| Positive control (PC): | 1% Triton X-100 in artificial saliva |
| Negative control (NC): | Artificial saliva |

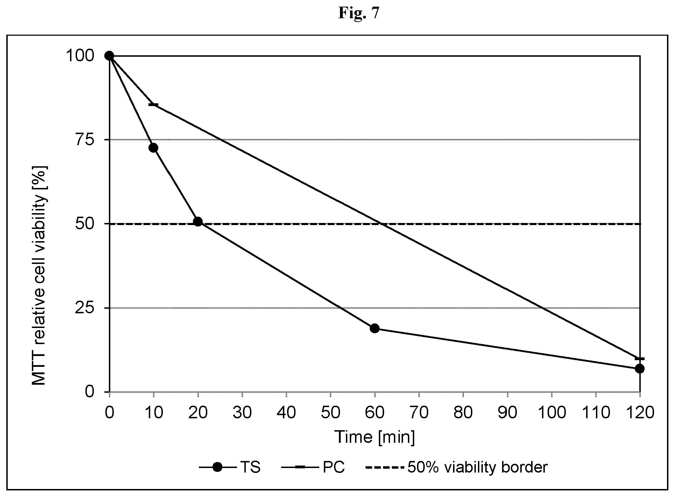

Relative cell viability is calculated for each tissue as % of the mean of the negative control tissues based on the OD readout value. Skin irritation potential of the test substance is predicted if the remaining relative cell viability is below 50%. The results are shown in Table 7.1 and Figure 7.

**Table 7.1**

| **MTT relative cell viability [%]** | | | |
|---|---|---|---|
| **Elapsed time [min]** | **TS (n = 6)** | **PC (n = 6)** | **NC (n = 6)** |
| **10** | 72.62 | 85.42 | - |
| **20** | 50.67 | - | - |
| **60** | 18.84 | - | 100.00 |
| **120** | 6.86 | 9.84 | - |

The cell viability experiments show that the specific enhancer combination (sodium caprate and sodium glycocholate) has non-critical skin irritation potential (50% viability border was reached after 20 min of exposure). The skin irritation potential can be further reduced by using a lower amount of enhancers.

### The invention relates in particular to the following further embodiments:

1. Transmucosal therapeutic system for the transmucosal administration of a GLP-1 receptor agonist comprising a mucoadhesive layer structure, said mucoadhesive layer structure comprising
   B) an active agent-containing layer comprising
   1. a GLP-1 receptor agonist;
   2. a combination of permeation enhancers; and
   3. a dissolvable film-forming agent, wherein
   the combination of permeation enhancers comprises
   iii) a first permeation enhancer selected from the group consisting of C6 to C12 fatty acids and salts thereof; and
   iv) a second permeation enhancer selected from the group consisting of bile acids and salts thereof.
2. Transmucosal therapeutic system according to embodiment 1, wherein the first permeation enhancer is selected from the group consisting of caproic acid, heptanoic acid, caprylic acid, nonanoic acid, capric acid, undecanoic acid, lauric acid and salts thereof.
3. Transmucosal therapeutic system according to embodiment 1 or 2, wherein the first permeation enhancer is selected from the group consisting of caproic acid, caprylic acid, capric acid, lauric acid and salts thereof.
4. Transmucosal therapeutic system according to any one of embodiments 1 to 3, wherein the first permeation enhancer is selected from the group consisting of sodium caproate, sodium caprylate, sodium caprate, sodium laurate, potassium caproate, potassium caprylate, potassium caprate, and potassium laurate.
5. Transmucosal therapeutic system according to any one of embodiments 1 to 4, wherein the first permeation enhancer is selected from the group consisting of sodium caprate and potassium caprate.
6. Transmucosal therapeutic system according to any one of embodiments 1 to 5, wherein the second permeation enhancer is selected from the group consisting of cholic acid, taurocholic acid, glycocholic acid, deoxycholic acid, taurodeoxycholic acid, glycodeoxycholic acid, chenodeoxycholic acid, taurochenodeoxycholic acid, glycochenodeoxycholic acid, and salts thereof.
7. Transmucosal therapeutic system according to any one of embodiments 1 to 6, wherein the second permeation enhancer is selected from the group consisting of sodium cholate, sodium taurocholate, sodium glycocholate, sodium deoxycholate, sodium taurodeoxycholate, sodium glycodeoxycholate, sodium ursodeoxycholate, sodium chenodeoxycholate, sodium taurochenodeoxycholate, sodium glycochenodeoxycholate, sodium cholylsarcosine and sodium N-methyl taurocholate.
8. Transmucosal therapeutic system according to any one of embodiments 1 to 7, wherein
   the second permeation enhancer is sodium glycocholate.
9. Transmucosal therapeutic system according to any one of embodiments 1 to 8, wherein
   if the first permeation enhancer is sodium caprate, the second permeation enhancer is not sodium chenodeoxycholate.
10. Transmucosal therapeutic system according to any one of embodiments 1 to 9, wherein
   if the first permeation enhancer is sodium caprate, the second permeation enhancer is selected from the group consisting of sodium cholate, sodium taurocholate, sodium glycocholate, sodium deoxycholate, sodium taurodeoxycholate, sodium glycodeoxycholate, sodium ursodeoxycholate, sodium taurochenodeoxycholate, sodium glycochenodeoxycholate, sodium cholylsarcosine and sodium N-methyl taurocholate.
11. Transmucosal therapeutic system according to any one of embodiments 1 to 10, wherein
   if the first permeation enhancer is sodium caprate, the second permeation enhancer is not sodium deoxycholate.
12. Transmucosal therapeutic system according to any one of embodiments 1 to 11, wherein
   if the first permeation enhancer is sodium caprate, the second permeation enhancer is selected from the group consisting of sodium cholate, sodium taurocholate, sodium glycocholate, sodium chenodeoxycholate, sodium taurodeoxycholate, sodium glycodeoxycholate, sodium ursodeoxycholate, sodium taurochenodeoxycholate, sodium glycochenodeoxycholate, sodium cholylsarcosine and sodium N-methyl taurocholate.
13. Transmucosal therapeutic system according to any one of embodiments 1 to 12, wherein
   the amount of the combination of permeation enhancers is from 6 wt-% to less than or equal to 60 wt-%, from 10 wt-% to less than or equal to 42 wt-%, or from 12 wt-% to less than or equal to 36 wt-% of the active agent-containing layer.
14. Transmucosal therapeutic system according to any one of embodiments 1 to 13, wherein
   the amount of the first permeation enhancer and/or the second permeation enhancer is from 3 wt-% to less than or equal to 30 wt-%, from 50 wt-% to less than or equal to 21 wt-%, or from 6 wt-% to less than or equal to 18 wt-% of the active agent-containing layer.
15. Transmucosal therapeutic system according to any one of embodiments 1 to 14, wherein
   the amount of the GLP-1 receptor agonist is at least 0.01 wt-%, at least 0.1 wt-%, at least 0.5 wt-%, at least 1 wt-%, or at least 2 wt-% of the active agent-containing layer.
16. Transmucosal therapeutic system according to any one of embodiments 1 to 15, wherein
   the amount of the GLP-1 receptor agonist is less than or equal to 25 wt-%, less than or equal to 20 wt-%, or less than or equal to 10 wt-% of the active agent-containing layer.
17. Transmucosal therapeutic system according to any one of embodiments 1 to 16, wherein
   the amount of GLP-1 receptor agonist is from 0.5 to less than or equal to 25 wt-%, from 1 to less than or equal to 20 wt-%, or from 2 to less than or equal to 10 wt-% of the active agent-containing layer.
18. Transmucosal therapeutic system according to any one of embodiments 1 to 17, wherein
   the GLP-1 receptor agonist is selected from the group consisting of albiglutide, beinaglutide, cotadutide, dulaglutide, efinopegdutide, efocipegtrutide, efpeglenatide, exenatide, liraglutide, lixisenatide, pemvidutide, retatrutide, semaglutide, survodutide, taspoglutide, and tirzepatide.
19. Transmucosal therapeutic system according to any one of embodiments 1 to 18, wherein
   the GLP-1 receptor agonist is selected from the group consisting of liraglutide, semaglutide and tirzepatide.
20. Transmucosal therapeutic system according to any one of embodiments 1 to 19, wherein
   the GLP-1 receptor agonist is semaglutide.
21. Transmucosal therapeutic system according to any one of embodiments 1 to 20, wherein
   the weight ratio of first permeation enhancer to GLP-1 receptor agonist is at least 0.3:1, at least 1:1, at least 2:1, or at least 3:1.
22. Transmucosal therapeutic system according to any one of embodiments 1 to 21, wherein
   the weight ratio of first permeation enhancer to GLP-1 receptor agonist is 100:1 or less, 70:1 or less, 50:1 or less, or 40:1 or less.
23. Transmucosal therapeutic system according to any one of embodiments 1 to 22, wherein
   the weight ratio of first permeation enhancer to GLP-1 receptor agonist is from 0.3:1 to 100:1, from 1:1 to 70:1, from 2:1 to 50:1, or from 3:1 to 40:1.
24. Transmucosal therapeutic system according to any one of embodiments 1 to 23, wherein
   the weight ratio of second permeation enhancer to GLP-1 receptor agonist is at least 1.5:1, at least 3:1, at least 10:1, or at least 20:1.
25. Transmucosal therapeutic system according to any one of embodiments 1 to 24, wherein
   the weight ratio of second permeation enhancer to GLP-1 receptor agonist is 100:1 or less, 70:1 or less, 50:1 or less, or 40:1 or less.
26. Transmucosal therapeutic system according to any one of embodiments 1 to 25, wherein
   the weight ratio of second permeation enhancer to GLP-1 receptor agonist is from 1.5:1 to 100:1, from 3:1 to 70:1, from 10:1 to 50:1, or from 20:1 to 40:1.
27. Transmucosal therapeutic system according to any one of embodiments 1 to 26, wherein
   the weight ratio of first permeation enhancer to second permeation enhancer is at least 1:2, or at least 1:1.
28. Transmucosal therapeutic system according to any one of embodiments 1 to 27, wherein
   the weight ratio of first permeation enhancer to second permeation enhancer is 10:1, or less or 6:1 or less.
29. Transmucosal therapeutic system according to any one of embodiments 1 to 28, wherein
   the weight ratio of first permeation enhancer to second permeation enhancer is from 1:2 to 10:1, or from 1:1 to 6:1.
30. Transmucosal therapeutic system according to any one of embodiments 1 to 29, wherein
   the weight ratio of first permeation enhancer to second permeation enhancer to GLP-1 receptor agonist is about 3:3:1, about 4:4:1, or about 5:5:1.
31. Transmucosal therapeutic system according to any one of embodiments 1 to 30, wherein
   the dissolvable film-forming agent, if casted into a film having an area weight of from 100 to 250 g/m², or of 200 g/m², dissolves in water, in artificial or natural saliva, or in any other aqueous medium, at 37 °C and 150 rpm, in less than 5 hours, less than 3 hours, less than 2 hours, or less than 1.5 hours, or in more than 10 minutes, more than 30 minutes, more than 45 minutes or more than 50 minutes, or more than 10 minutes and less than 5 hours, in more than 30 minutes and less than 3 hours, more than 45 minutes and less than 2 hours, or in more than 50 minutes and less than 1.5 hours.
32. Transmucosal therapeutic system according to any one of embodiments 1 to 31, wherein
   the dissolvable film-forming agent is selected from the group consisting of polymers such as polyvinylpyrrolidone, methyl cellulose, ethyl cellulose, hydroxyethyl cellulose, hydroxypropyl cellulose, hydroxypropyl methyl cellulose, carboxymethyl cellulose sodium, polyethylene glycol- polyvinyl acetate- and polyvinylcaprolactame-based graft copolymers, polyvinyl alcohol, polyvinyl alcohol-polyethylene glycol copolymers, polyvinylpyrrolidone-polyvinylacetate copolymers, polyethylene oxides, polyethylene glycols, methacrylic acid - methyl methacrylate copolymers, and methacrylic acid - ethyl methacrylate copolymers, and natural film-forming agents such as shellac, pectin, gelatine, alginate, pullulan and starch derivatives, and any mixtures thereof.
33. Transmucosal therapeutic system according to any one of embodiments 1 to 32, wherein
   the dissolvable film-forming agent is selected from the group consisting of polymers such as polyvinylpyrrolidone, methyl cellulose, hydroxyethyl cellulose, hydroxypropyl cellulose, ,hydroxypropyl methyl cellulose, carboxymethyl cellulose sodium, polyethylene glycol-polyvinyl acetate- and polyvinylcaprolactame-based graft copolymers, polyvinyl alcohol, polyvinyl alcohol-polyethylene glycol copolymers, polyvinylpyrrolidone-polyvinylacetate copolymers, polyethylene oxides, polyethylene glycols, and any mixtures thereof.
34. Transmucosal therapeutic system according to any one of embodiments 1 to 33, wherein
   the dissolvable film-forming agent is selected from the group consisting of polymers such as methyl cellulose, hydroxyethyl cellulose, hydroxypropyl cellulose, hydroxypropyl methyl cellulose, carboxymethyl cellulose sodium, and any mixtures thereof.
35. Transmucosal therapeutic system according to any one of embodiments 1 to 34, wherein
   the dissolvable film-forming agent comprises a hydroxypropyl methyl cellulose or a mixture of hydroxypropyl methyl celluloses.
36. Transmucosal therapeutic system according to any one of embodiments 1 to 35, wherein
   the dissolvable film-forming agent comprises a hydroxypropyl methyl cellulose with a methoxyl content of from 10 % to less than or equal to 40 %, and/or a hydroxypropoxyl content of from 2 % to less than or equal to 17 %, from 5 % to less than or equal to 14 %, or from 7 % to less than or equal to 12 %.
37. Transmucosal therapeutic system according to any one of embodiments 1 to 36, wherein
   the dissolvable film-forming agent comprises a hydroxypropyl methyl cellulose having a viscosity of 10 mPa s or less, 5 mPa s or less, or 3 mPa s or less, and/or
   the dissolvable film-forming agent comprises a hydroxypropyl methyl cellulose having a viscosity of at least 10 mPa s, at least 50 mPa s, or at least 80 mPa s.
38. Transmucosal therapeutic system according to any one of embodiments 1 to 37, wherein
   the dissolvable film-forming agent comprises a mixture of a first hydroxypropyl methyl cellulose having a viscosity of from 1.5 mPa s to 5 mPa s or less, from 2 mPa s to 4 mPa s or less, or from 2.4 mPa s to 3.6 mPa s or less, and a second hydroxypropyl cellulose having a viscosity of from 10 mPa s to 20 mPa s or less, from 18.5 mPa s to 11.5 mPa s or less, or from 12 mPa s to 18 mPa s or less.
39. Transmucosal therapeutic system according to any one of embodiments 1 to 38, wherein
   the dissolvable film-forming agent comprises a mixture of a first hydroxypropyl methyl cellulose having a viscosity of from 1.5 mPa s to 5 mPa s or less, from 2 mPa s to 4 mPa s or less, or from 2.4 mPa s to 3.6 mPa s or less, and a second hydroxypropyl cellulose having a viscosity of from 10 mPa s to 200 mPa s or less, from 50 mPa s to 150 mPa s or less, or from 80 mPa s to 120 mPa s or less.
40. Transmucosal therapeutic system according to any one of embodiments 1 to 39, wherein
   the dissolvable film-forming agent comprises a mixture of a first hydroxypropyl methyl cellulose having a viscosity of from 1.5 mPa s to 5 mPa s or less, from 2 mPa s to 4 mPa s or less, or from 2.4 mPa s to 3.6 mPa s or less, a second hydroxypropyl cellulose having a viscosity of from 10 mPa s to 200 mPa s or less, from 50 mPa s to 150 mPa s or less, or from 80 mPa s to 120 mPa s or less, and a third hydroxypropyl methyl cellulose having a viscosity of from 10,000 mPa s to 250,000 mPa s, from 50,000 mPa s to 180,000 mPa s, or from 75,000 mPa s to 140,000 mPa s.
41. Transmucosal therapeutic system according to any one of embodiments 1 to 40, wherein
   the dissolvable film-forming agent comprises a mixture of a first hydroxypropyl methyl cellulose having a viscosity of about 3 mPa s, a second hydroxypropyl methyl cellulose having a viscosity of about 100 mPa s, and a third hydroxypropyl methyl having a viscosity of about 100,000 mPa s.
42. Transmucosal therapeutic system according to any one of embodiments 1 to 41, wherein
   the amount of the dissolvable film-forming agent is at least 25 wt-%, at least 30 wt-% or at least 40 wt-%, or the amount of the dissolvable film-forming agent is less than or equal to 75 wt-%, less than or equal to 70 wt-% or less than or equal to 60 wt-%, or the amount of the dissolvable film-forming agent ranges from 25 to 75 wt-%, from 30 to 70 wt-%, or from 40 to 60 wt-% of the active agent-containing layer.
43. Transmucosal therapeutic system according to any one of embodiments 1 to 42, wherein the active agent-containing layer further comprises basal insulin.
44. Transmucosal therapeutic system according to any one of embodiments 1 to 43, wherein
   the active agent-containing layer further comprises one or more excipients selected from the group consisting of taste masking agents, sweeteners, flavoring agents, colorants, solubilizers, plasticizers, humectants, disintegrants, emulsifiers, antioxidants, stabilizers, buffer reagents, further permeation enhancers, and further film-forming agents.
45. Transmucosal therapeutic system according to any one of embodiments 1 to 44, wherein
   the active agent-containing layer further comprises one or more excipients selected from the group consisting of taste masking agents, sweeteners, and flavoring agents.
46. Transmucosal therapeutic system according to any one of embodiments 1 to 45, wherein
   the active agent-containing layer comprises one or more natural or artificial taste masking agents selected from the group consisting of flavor suppressing agents, bitter masking agents, sour masking agents and salty masking agents.
47. Transmucosal therapeutic system according to embodiment 48, wherein
   the active agent-containing layer comprises the one or more natural or artificial taste masking agents in an amount of at least 0.6 wt-%, at least 1.2 wt-% or at least 1.5 wt-%, or in an amount of less than or equal to 6.0 wt-%, less than or equal to 4.0 wt-% or less than or equal to 3.0 wt-%, or in an amount of from 0.6 to 6.0 wt-%, from 1.2 to 4.0 wt-%, or from 1.5 to 3.0 wt-% each.
48. Transmucosal therapeutic system according to any one of embodiments 1 to 47, wherein
   the active agent-containing layer comprises one or more natural or artificial sweeteners selected from the group consisting of saccharose, glucose, fructose, sorbitol, mannitol, isomalt maltitol lactitol, xylitol, erythritol, sucralose, acesulfame potassium, aspartame, cyclamate, neohesperidine, neotame, steviol glycosides, thaumatin and saccharin sodium.
49. Transmucosal therapeutic system according to embodiment 48, wherein
   the active agent-containing layer comprises the one or more natural or artificial sweeteners in an amount of at least 0.05 wt-%, at least 0.1 wt-% or at least 0.15 wt-%, or in an amount of less than or equal to 2.0 wt-%, less than or equal to 1.5 wt-% or less than or equal to 1.0 wt-%, or in an amount of from 0.05 to 2.0 wt-%, from 0.1 to 1.5 wt-%, or from 0.15 to 1.0 wt-% each.
50. Transmucosal therapeutic system according to any one of embodiments 1 to 49, wherein
   the active agent-containing layer comprises one or more natural or artificial flavoring agents selected from the group consisting of vanillin, methyl salicylate, menthol, manzanate, diacetyl, acetylpropionyl, acetoin, isoamyl acetate, benzaldehyde, cinnamaldehyde, ethyl propionate, methyl anthranilate, limonene, ethyl decadienoate, allyl hexanoate, ethyl maltol, 2,4-dithiapentane, ethylvanillin and eucalyptol as well as flavoring compositions such as peppermint flavor.
51. Transmucosal therapeutic system according to embodiment 50, wherein
   the active agent-containing layer comprises the one or more flavoring agents in an amount of at least 0.3 wt-%, at least 0.6 wt-% or at least 0.8 wt-%, or in an amount of less than or equal to 12 wt-%, less than or equal to 8 wt-%, or less than or equal to 6 wt-%, or in an amount of from 0.3 to 12 wt-%, from 0.6 to 8 wt-%, or from 0.8 to 6 wt-% each.
52. Transmucosal therapeutic system according to any one of embodiments 1 to 51, wherein
   the active agent-containing layer comprises substantially no water.
53. Transmucosal therapeutic system according to any one of embodiments 1 to 52, wherein
   the active agent-containing layer comprises less than or equal to 12 wt-%, less than or equal to 8 wt-%, less than or equal to 5 wt-%, or less than or equal to 4 wt-% water.
54. Transmucosal therapeutic system according to any one of embodiments 1 to 53, wherein
   the active agent-containing layer is obtainable by drying a coated coating composition comprising the GLP-1 receptor agonist, the combination of enhancers, the dissolvable film-forming agent, and water.
55. Transmucosal therapeutic system according to any one of embodiments 1 to 54, wherein
   the active agent-containing layer has an area weight of at least 40 g/m², at least 80 g/m², or at least 100 g/m², or has an area weight of less than or equal to 500 g/m², less than or equal to 300 g/m², or less than or equal to 250 g/m², or has an area weight of from 50 to 500 g/m², from 100 to 300 g/m², or from 120 to 25 g/m².
56. Transmucosal therapeutic system according to any one of embodiments 1 to 55, wherein
   the active agent-containing layer comprises at least 0.01 mg/cm², at least 0.05 mg/cm², at least 0.1 mg/cm², at least 0.2 mg/cm², or at least 0.4 mg/cm² GLP-1 receptor agonist, or the active agent-containing layer comprises less than or equal to 2.0 mg/cm², less than or equal to 1.2 mg/cm², or less than or equal to 1.0 mg/cm² GLP-1 receptor agonist, or the active agent-containing layer comprises from 0.1 to 2.0 mg/cm², from 0.2 to 1.2 mg/cm², or from 0.4 to 1.0 mg/cm² GLP-1 receptor agonist.
57. Transmucosal therapeutic system according to any one of embodiments 1 to 56, wherein
   the mucoadhesive layer structure further comprises one or more further layers selected from
   A) a mucosa-contacting layer, and
   B) a cosmetic layer,
   wherein
   the further layers adjoin the active agent-containing layer, and
   the mucosa-contacting layer and the cosmetic layer, if both are present, adjoin the active agent-containing layer on opposite sides.
58. Transmucosal therapeutic system according to any one of embodiments 1 to 57, wherein
   the mucoadhesive layer structure does not comprise a mucosa-contacting layer.
59. Transmucosal therapeutic system according to any one of embodiments 1 to 58, wherein
   the mucoadhesive layer structure further comprises a mucosa-contacting layer and the mucosa-contacting layer is mucoadhesive.
60. Transmucosal therapeutic system according to any one of embodiments 1 to 59, wherein
   the mucoadhesive layer structure consists of the active agent-containing layer.
61. Transmucosal therapeutic system according to any one of embodiments 1 to 60, wherein
   the active agent-containing layer is mucoadhesive.
62. Transmucosal therapeutic system according to any one of embodiments 1 to 61, wherein
   the mucoadhesive layer structure contains GLP-1 receptor agonist in a therapeutically effective amount.
63. Transmucosal therapeutic system according to any one of embodiments 1 to 62, wherein
   the mucoadhesive layer structure comprises at least 0.1 mg, at least 0.2 mg, or at least 0.4 mg GLP-1 receptor agonist, or the mucoadhesive layer structure comprises less than or equal to 20 mg, less than or equal to 15 mg, or less than or equal to 10 mg GLP-1 receptor agonist, or the mucoadhesive layer structure comprises from 0.1 mg to 20 mg, from 0.2 mg to 15 mg, or from 0.4 mg to 10 mg GLP-1 receptor agonist.
64. Transmucosal therapeutic system according to any one of embodiments 1 to 63, wherein
   the transmucosal therapeutic system has an area of release of at least 0.1 cm², at least 0.2 cm², or at least 0.5 cm², or has an area of release of less than or equal to 10 cm², less than or equal to 7 cm², or less than or equal to 5 cm², or has an area of release of from 0.1 to 10 cm², from 0.2 to 7 cm², or from 0.5 to 5 cm².
65. Transmucosal therapeutic system according to any one of embodiments 1 to 64, wherein
   the transmucosal therapeutic system does not comprise a backing layer.
66. Transmucosal therapeutic system according to any one of embodiments 1 to 65, further comprising a release liner.
67. Transmucosal therapeutic system according to any one of embodiments 1 to 66, wherein
   the transmucosal therapeutic system is in the form of a film.
68. Transmucosal therapeutic system according to any one of embodiments 1 to 67, wherein
   the transmucosal therapeutic system is in the form of a thin film having an area weight of at least 40 g/m², at least 80 g/m², or at least 100 g/m², or an area weight of less than or equal to 500 g/m², less than or equal to 300 g/m², or less than or equal to 250 g/m², or an area weight of from 40 to 500 g/m², from 80 to 300 g/m², or from 100 to 250 g/m².
69. Transmucosal therapeutic system according to any one of embodiments 1 to 68, wherein
   the transmucosal therapeutic system is in the form of a film having a circular, rectangular or square shape.
70. Transmucosal therapeutic system according to any one of embodiments 1 to 69, wherein
   the transmucosal therapeutic system provides a cumulative release of GLP-1 receptor agonist as measured with reconstructed human epithelial tissue of
   at least 0.002 mg/cm², at least 0.05 mg/cm² or at least 0.008 mg/cm², or less than or equal to 0.04 mg/cm², less than or equal to 0.03 mg/cm², or less than or equal to 0.025 mg/cm², or of from 0.002 mg/cm² to 0.04 mg/cm², from 0.005 mg/cm² to 0.03 mg/cm², or from 0.008 mg/cm² to 0.025 mg/cm² over a time period of 1 hour, and/or
   at least 0.005 mg/cm², at least 0.01 mg/cm² or at least 0.02 mg/cm², or less than or equal to 0.15 mg/cm², less than or equal to 0.1 mg/cm², or less than or equal to 0.07 mg/cm², or of from 0.005 mg/cm² to 0.15 mg/cm², from 0.01 mg/cm² to 0.1 mg/cm², or from 0.02 mg/cm² to 0.07 mg/cm² over a time period of 2 hours, and/or
   at least 0.01 mg/cm², at least 0.02 mg/cm² or at least 0.03 mg/cm², or less than or equal to 0.3 mg/cm², less than or equal to 0.2 mg/cm², or less than or equal to 0.1 mg/cm², or of from 0.01 mg/cm² to 0.3 mg/cm², from 0.02 mg/cm² to 0.2 mg/cm², or from 0.03 mg/cm² to 0.1 mg/cm² over a time period of 3 hours, and/or
   at least 0.02 mg/cm², at least 0.05 mg/cm² or at least 0.08 mg/cm², or less than or equal to 0.4 mg/cm², less than or equal to 0.3 mg/cm², or less than or equal to 0.25 mg/cm², or of from 0.02 mg/cm² to 0.4 mg/cm², from 0.05 mg/cm² to 0.3 mg/cm², or from 0.08 mg/cm² to 0.25 mg/cm² over a time period of 5 hours.
71. Transmucosal therapeutic system according to any one of embodiments 1 to 70, for use in a method of treating a human patient.
72. Transmucosal therapeutic system according to any one of embodiments 1 to 70, for use in a method of treating diabetes and/or obesity.
73. Transmucosal therapeutic system according to any one of embodiments 1 to 70, for use in a method of treating type 2 diabetes mellitus.
74. Transmucosal therapeutic system according to any one of embodiments 1 to 70, for use in a method of supporting weight management.
75. Transmucosal therapeutic system for use according to any one of embodiments 1 to 70, wherein
   the transmucosal therapeutic system is administered by applying the mucoadhesive layer structure to the mucosa, in particular to the buccal, sublingual, gingival or palatal mucosa, of the oral cavity of a human patient and maintained on the mucosa until dissolved.
76. Use of a transmucosal therapeutic system according to any one of embodiments 1 to 70, in the manufacture of a medicament for treating a human patient.
77. Method of treatment, wherein
   the transmucosal therapeutic system according to any one of embodiments 1 to 70 is administered to a human patient.
78. Process of manufacture of an active agent-containing layer of a transmucosal therapeutic system according to any one of embodiments 1 to 70, comprising the steps of:
   (a) combining at least the GLP-1 receptor agonist, the combination of permeation enhancers and the dissolvable film-forming agent in a solvent to obtain a coating composition;
   (b) coating the coating composition onto a release liner; and
   (c) drying the coated coating composition to form the active agent-containing layer.
79. Transmucosal therapeutic system for the transmucosal administration of a GLP-1 receptor agonist comprising a mucoadhesive layer structure, said mucoadhesive layer structure comprising at least
   B) an active agent-containing layer comprising
   1. a GLP-1 receptor agonist in an amount of from 2 wt-% to less than or equal to 10 wt-%;
   2. a combination of permeation enhancers in an amount of from 12 wt-% to less than or equal to 36 wt-%; and
   3. a dissolvable film-forming agent in an amount of from 40 wt-% to less than or equal to 60 wt-%, wherein
   the combination of permeation enhancers comprises
   iii) a first permeation enhancer selected from the group consisting of C6 to C12 fatty acids and salts thereof; and
   iv) a second permeation enhancer selected from the group consisting of bile acids and salts thereof, and
   the dissolvable film-forming agent is a hydroxypropyl methyl cellulose.

## Claims

1. Transmucosal therapeutic system for the transmucosal administration of a GLP-1 receptor agonist comprising a mucoadhesive layer structure, said mucoadhesive layer structure comprising
an active agent-containing layer comprising
1. a GLP-1 receptor agonist;
2. a combination of permeation enhancers; and
3. a dissolvable film-forming agent, wherein
the combination of permeation enhancers comprises
i) a first permeation enhancer selected from the group consisting of C6 to C12 fatty acids and salts thereof; and
ii) a second permeation enhancer selected from the group consisting of bile acids and salts thereof.

2. Transmucosal therapeutic system according to claim 1, wherein
the first permeation enhancer is selected from the group consisting of caproic acid, heptanoic acid, caprylic acid, nonanoic acid, capric acid, undecanoic acid, lauric acid and salts thereof, or
the first permeation enhancer is selected from the group consisting of caproic acid, caprylic acid, capric acid, lauric acid and salts thereof, or
the first permeation enhancer is selected from the group consisting of sodium caproate, sodium caprylate, sodium caprate, sodium laurate, potassium caproate, potassium caprylate, potassium caprate, and potassium laurate, or
the first permeation enhancer is selected from the group consisting of sodium caprate and potassium caprate.

3. Transmucosal therapeutic system according to claim 1 or 2, wherein
the second permeation enhancer is selected from the group consisting of cholic acid, taurocholic acid, glycocholic acid, deoxycholic acid, taurodeoxycholic acid, glycodeoxycholic acid, chenodeoxycholic acid, taurochenodeoxycholic acid, glycochenodeoxycholic acid, and salts thereof, or
the second permeation enhancer is selected from the group consisting of sodium cholate, sodium taurocholate, sodium glycocholate, sodium deoxycholate, sodium taurodeoxycholate, sodium glycodeoxycholate, sodium ursodeoxycholate, sodium chenodeoxycholate, sodium taurochenodeoxycholate, sodium glycochenodeoxycholate, sodium cholylsarcosine and sodium N-methyl taurocholate, or
the second permeation enhancer is sodium glycocholate.

4. Transmucosal therapeutic system according to any one of claims 1 to 3, wherein
the amount of the combination of permeation enhancers is from 6 wt-% to less than or equal to 60 wt-%, from 10 wt-% to less than or equal to 42 wt-%, or from 12 wt-% to less than or equal to 36 wt-% of the active agent-containing layer, and/or
the amount of the first permeation enhancer and/or the second permeation enhancer is from 3 wt-% to less than or equal to 30 wt-%, from 50 wt-% to less than or equal to 21 wt-%, or from 6 wt-% to less than or equal to 18 wt-% of the active agent-containing layer.

5. Transmucosal therapeutic system according to any one of claims 1 to 4, wherein
the amount of the GLP-1 receptor agonist is at least 0.01 wt-%, at least 0.1 wt-%, at least 0.5 wt-%, at least 1 wt-%, or at least 2 wt-% of the active agent-containing layer, and/or less than or equal to 25 wt-%, less than or equal to 20 wt-%, or less than or equal to 10 wt-% of the active agent-containing layer, or the amount of GLP-1 receptor agonist is from 0.5 to less than or equal to 25 wt-%, from 1 to less than or equal to 20 wt-%, or from 2 to less than or equal to 10 wt-% of the active agent-containing layer.

6. Transmucosal therapeutic system according to any one of claims 1 to 5, wherein
the GLP-1 receptor agonist is selected from the group consisting of albiglutide, beinaglutide, cotadutide, dulaglutide, efinopegdutide, efocipegtrutide, efpeglenatide, exenatide, liraglutide, lixisenatide, pemvidutide, retatrutide, semaglutide, survodutide, taspoglutide, and tirzepatide, or
the GLP-1 receptor agonist is selected from the group consisting of liraglutide, semaglutide and tirzepatide, or
the GLP-1 receptor agonist is semaglutide.

7. Transmucosal therapeutic system according to any one of claims 1 to 6, wherein
the weight ratio of first permeation enhancer to GLP-1 receptor agonist is at least 0.3:1, at least 1:1, at least 2:1, or at least 3:1, and/or 100:1 or less, 70:1 or less, 50:1 or less, or 40:1 or less, or the weight ratio of first permeation enhancer to GLP-1 receptor agonist is from 0.3:1 to 100:1, from 1:1 to 70:1, from 2:1 to 50:1, or from 3:1 to 40:1, and/or
the weight ratio of second permeation enhancer to GLP-1 receptor agonist is at least 1.5:1, at least 3:1, at least 10:1, or at least 20:1, and/or 100:1 or less, 70:1 or less, 50:1 or less, or 40:1 or less, or the weight ratio of second permeation enhancer to active agent is from 1.5:1 to 100:1, from 3:1 to 70:1, from 10:1 to 50:1, or from 20:1 to 40:1.

8. Transmucosal therapeutic system according to any one of claims 1 to 7, wherein
the weight ratio of first permeation enhancer to second permeation enhancer is at least 1:2, or at least 1:1, and/or 10:1, or less or 6:1 or less, or the weight ratio of first permeation enhancer to second permeation enhancer is from 1:2 to 10:1, or from 1:1 to 6:1, and/or
the weight ratio of first permeation enhancer to second permeation enhancer to GLP-1 receptor agonist is about 3:3:1, about 4:4:1, or about 5:5:1.

9. Transmucosal therapeutic system according to any one of claims 1 to 8, wherein
the dissolvable film-forming agent is selected from the group consisting of polymers such as polyvinylpyrrolidone, methyl cellulose, ethyl cellulose, hydroxyethyl cellulose, hydroxypropyl cellulose, hydroxypropyl methyl cellulose, carboxymethyl cellulose sodium, polyethylene glycol- polyvinyl acetate- and polyvinylcaprolactame-based graft copolymers, polyvinyl alcohol, polyvinyl alcohol-polyethylene glycol copolymers, polyvinylpyrrolidone-polyvinylacetate copolymers, polyethylene oxides, polyethylene glycols, methacrylic acid - methyl methacrylate copolymers, and methacrylic acid - ethyl methacrylate copolymers, and natural film-forming agents such as shellac, pectin, gelatine, alginate, pullulan and starch derivatives, and any mixtures thereof, or
the dissolvable film-forming agent is selected from the group consisting of polymers such as polyvinylpyrrolidone, methyl cellulose, hydroxyethyl cellulose, hydroxypropyl cellulose, hydroxypropyl methyl cellulose, carboxymethyl cellulose sodium, polyethylene glycol-polyvinyl acetate- and polyvinylcaprolactame-based graft copolymers, polyvinyl alcohol, polyvinyl alcohol-polyethylene glycol copolymers, polyvinylpyrrolidone-polyvinylacetate copolymers, polyethylene oxides, polyethylene glycols, and any mixtures thereof, or
the dissolvable film-forming agent is selected from the group consisting of polymers such as methyl cellulose, hydroxyethyl cellulose, hydroxypropyl cellulose, hydroxypropyl methyl cellulose, carboxymethyl cellulose sodium, and any mixtures thereof, or
the dissolvable film-forming agent comprises a hydroxypropyl methyl cellulose or a mixture of hydroxypropyl methyl celluloses.

10. Transmucosal therapeutic system according to any one of claims 1 to 9, wherein
the dissolvable film-forming agent comprises a hydroxypropyl methyl cellulose with a methoxyl content of from 10 % to less than or equal to 40 %, and/or a hydroxypropoxyl content of from 2 % to less than or equal to 17 %, from 5 % to less than or equal to 14 %, or from 7 % to less than or equal to 12 %, or
the dissolvable film-forming agent comprises a hydroxypropyl methyl cellulose having a viscosity of 10 mPa s or less, 5 mPa s or less, or 3 mPa s or less, and/or a hydroxypropyl methyl cellulose having a viscosity of at least 10 mPa s, at least 50 mPa s, or at least 80 mPa s, or
the dissolvable film-forming agent comprises a mixture of a first hydroxypropyl methyl cellulose having a viscosity of from 1.5 mPa s to 5 mPa s or less, from 2 mPa s to 4 mPa s or less, or from 2.4 mPa s to 3.6 mPa s or less, and a second hydroxypropyl cellulose having a viscosity of from 10 mPa s to 20 mPa s or less, from 18.5 mPa s to 11.5 mPa s or less, or from 12 mPa s to 18 mPa s or less, or a mixture of a first hydroxypropyl methyl cellulose having a viscosity of from 1.5 mPa s to 5 mPa s or less, from 2 mPa s to 4 mPa s or less, or from 2.4 mPa s to 3.6 mPa s or less, and a second hydroxypropyl cellulose having a viscosity of from 10 mPa s to 200 mPa s or less, from 50 mPa s to 150 mPa s or less, or from 80 mPa s to 120 mPa s or less, or a mixture of a first hydroxypropyl methyl cellulose having a viscosity of from 1.5 mPa s to 5 mPa s or less, from 2 mPa s to 4 mPa s or less, or from 2.4 mPa s to 3.6 mPa s or less, a second hydroxypropyl cellulose having a viscosity of from 10 mPa s to 200 mPa s or less, from 50 mPa s to 150 mPa s or less, or from 80 mPa s to 120 mPa s or less, and a third hydroxypropyl methyl cellulose having a viscosity of from 10,000 mPa s to 250,000 mPa s, from 50,000 mPa s to 180,000 mPa s, or from 75,000 mPa s to 140,000 mPa s.

11. Transmucosal therapeutic system according to any one of claims 1 to 10, wherein
the amount of the dissolvable film-forming agent is at least 25 wt-%, at least 30 wt-% or at least 40 wt-%, or the amount of the dissolvable film-forming agent is less than or equal to 75 wt-%, less than or equal to 70 wt-% or less than or equal to 60 wt-%, or the amount of the dissolvable film-forming agent ranges from 25 to 75 wt-%, from 30 to 70 wt-%, or from 40 to 60 wt-% of the active agent-containing layer.

12. Transmucosal therapeutic system according to any one of claims 1 to 11, wherein
the active agent-containing layer further comprises one or more excipients selected from the group consisting of taste masking agents, sweeteners, flavoring agents, colorants, solubilizers, plasticizers, humectants, disintegrants, emulsifiers, antioxidants, stabilizers, buffer reagents, further permeation enhancers, and further film-forming agents, or
the active agent-containing layer further comprises one or more excipients selected from the group consisting of taste masking agents, sweeteners, and flavoring agents.

13. Transmucosal therapeutic system according to any one of claims 1 to 12, wherein
the transmucosal therapeutic system provides a cumulative release of GLP-1 receptor agonist as measured with reconstructed human epithelial tissue of
at least 0.002 mg/cm², at least 0.05 mg/cm² or at least 0.008 mg/cm², or less than or equal to 0.04 mg/cm², less than or equal to 0.03 mg/cm², or less than or equal to 0.025 mg/cm², or of from 0.002 mg/cm² to 0.04 mg/cm², from 0.005 mg/cm² to 0.03 mg/cm², or from 0.008 mg/cm² to 0.025 mg/cm² over a time period of 1 hour, and/or
at least 0.005 mg/cm², at least 0.01 mg/cm² or at least 0.02 mg/cm², or less than or equal to 0.15 mg/cm², less than or equal to 0.1 mg/cm², or less than or equal to 0.07 mg/cm², or of from 0.005 mg/cm² to 0.15 mg/cm², from 0.01 mg/cm² to 0.1 mg/cm², or from 0.02 mg/cm² to 0.07 mg/cm² over a time period of 2 hours, and/or
at least 0.01 mg/cm², at least 0.02 mg/cm² or at least 0.03 mg/cm², or less than or equal to 0.3 mg/cm², less than or equal to 0.2 mg/cm², or less than or equal to 0.1 mg/cm², or of from 0.01 mg/cm² to 0.3 mg/cm², from 0.02 mg/cm² to 0.2 mg/cm², or from 0.03 mg/cm² to 0.1 mg/cm² over a time period of 3 hours, and/or
at least 0.02 mg/cm², at least 0.05 mg/cm² or at least 0.08 mg/cm², or less than or equal to 0.4 mg/cm², less than or equal to 0.3 mg/cm², or less than or equal to 0.25 mg/cm², or of from 0.02 mg/cm² to 0.4 mg/cm², from 0.05 mg/cm² to 0.3 mg/cm², or from 0.08 mg/cm² to 0.25 mg/cm² over a time period of 5 hours.

14. Transmucosal therapeutic system according to any one of claims 1 to 13,
for use in a method of treating a human patient, or in a method of treating diabetes and/or obesity, in particular in a method of treating type 2 diabetes mellitus and/or in a method of supporting weight management.

15. Process of manufacture of an active agent-containing layer of a transmucosal therapeutic system according to any one of claims 1 to 13, comprising the steps of:
(a) combining at least the GLP-1 receptor agonist, the combination of permeation enhancers and the dissolvable film-forming agent in a solvent to obtain a coating composition;
(b) coating the coating composition onto a release liner; and
(c) drying the coated coating composition to form the active agent-containing layer.
